# FASCICULE DE BREVET EUROPEEN

(11) **EP 3 158 071 B1**
(45) Date de publication et mention de la délivrance du brevet: **19.05.2021**
(21) Numéro de dépôt: 15732868.3
(22) Date de dépôt: 12.06.2015
(51) Int. Cl.: C12N 15/80

(54) **VARIANTS D'EXOGLUCANASES A ACTIVITE AMELIOREE ET LEURS UTILISATIONS**
VARIANTEN VON EXOGLUCANASEN MIT VERBESSERTER AKTIVITÄT UND VERWENDUNGEN DAVON
VARIANTS OF EXOGLUCANASES HAVING IMPROVED ACTIVITY AND USES THEREOF

(30) Priorité: 20.06.2014 FR 1455700
(43) Date de publication de la demande: 26.04.2017
(73) Titulaire: IFP Energies nouvelles, 92500 Rueil-Malmaison (FR); Protéus, 91160 Longjumeau (FR); CENTRE NATIONAL DE LA RECHERCHE SCIENTIFIQUE -CNRS-, 75016 Paris (FR)
(72) Inventeur: PERSILLON, Cécile, 30000 Nîmes (FR); ULLMANN, Christophe, 30000 Nîmes (FR); AYRINHAC, Céline, 30350 Domessargues (FR); BONZOM, Olivier, 30000 Nîmes (FR); FORT, Sébastien, 38410 Vaulnaveys-le-Haut (FR); ARMAND, Sylvie, 38000 Grenoble (FR); PRADEAU, Stéphanie, 38350 Saint-Honoré (FR); MARGEOT, Antoine, 75018 Paris (FR); ROUSSEAU, Lionel, 78440 Issou (FR); LE ROUX, Françoise, 92500 Rueil Malmaison (FR)
(74) Mandataire: Plasseraud IP
(86) Numéro de dépôt international: PCT/FR2015/051556
(87) Numéro de publication internationale: WO 2015/193587

(56) Documents cités:
- WO-A1-2012/068509
- WO-A1-2012/068509
- FR-A1- 2 782 323
- US-A1- 2008 167 214
- US-A1- 2008 167 214
- DATABASE Geneseq [Online] 19 juin 2014 (2014-06-19), "Hypocrea jecorina CBH1 polypeptide, SEQ ID 2.", XP002732689, extrait de EBI accession no. GSP:BBI75603 Database accession no. BBI75603 -& WO 2014/093294 A1 (DANISCO US INC [US]) 19 juin 2014 (2014-06-19)
- DATABASE Geneseq [Online] 19 juin 2014 (2014-06-19), "Hypocrea jecorina cellobiohydrolase 1 (CBH1) protein, SEQ ID 2.", XP002732690, extrait de EBI accession no. GSP:BBI74548 Database accession no. BBI74548
- DATABASE Geneseq [Online] 13 mars 2014 (2014-03-13), "Trichoderma reesei cellobiohydrolase I, SEQ ID 34.", XP002732691, extrait de EBI accession no. GSP:BBB73564 Database accession no. BBB73564 -& US 2014/017737 A1 (WOGULIS MARK [US]) 16 janvier 2014 (2014-01-16)
- DATABASE Geneseq [Online] 18 juillet 2013 (2013-07-18), "T. reesei mutant Cel7A protein, Y371C.", XP002732692, extrait de EBI accession no. GSP:BAO94743 Database accession no. BAO94743 -& US 2013/130353 A1 (TETER SARAH [US] ET AL) 23 mai 2013 (2013-05-23)
- DATABASE Geneseq [Online] 19 février 2009 (2009-02-19), "Trichoderma reesei cellobiohydrolase I protein, SEQ: 88.", XP002732693, extrait de EBI accession no. GSP:AUP68912 Database accession no. AUP68912

## Description

La possibilité de produire de l'éthanol à partir de la cellulose a reçu beaucoup d'attention en raison de la disponibilité de grandes quantités de matière première ainsi que de l'intérêt de l'éthanol à titre de carburant. Les matières premières naturelles cellulosiques pour un tel processus sont désignées par le terme "biomasse". De nombreux types de biomasse, par exemple le bois, les résidus agricoles, les cultures herbacées et les déchets solides municipaux, ont été considérés comme des matières premières potentielles pour la production de biocarburant. Ces matières sont constituées principalement de cellulose, d'hémicellulose et de lignine.

La cellulose est un polymère constitué de molécules de glucose reliées par des liens beta 1-4, qui sont très résistants à la dégradation ou à la dépolymérisation. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence d'acides concentrés ou dilués. Cependant, plusieurs inconvénients tels que la mauvaise récupération de l'acide lors de l'utilisation d'acides concentrés et la faible production de glucose dans le cas de l'utilisation d'acides dilués nuisent à l'économie du processus d'hydrolyse acide.

Pour surmonter les inconvénients du processus d'hydrolyse acide, les processus de conversion de la cellulose ont porté plus récemment sur l'hydrolyse enzymatique, à l'aide d'enzymes de type cellulase. Cette hydrolyse enzymatique de la biomasse lignocellulosique (par exemple, la cellulose) présente cependant l'inconvénient d'être un procédé industriel coûteux. De ce fait, il est nécessaire d'utiliser des souches de microorganismes sécréteurs de cellulases de plus en plus performantes. À ce titre, beaucoup de microorganismes comportent des enzymes qui hydrolysent la cellulose, tels que les champignons *Trichoderma, Aspergillus, Humicola, Fusarium* ainsi que des bactéries telles que *Thermomonospora, Bacillus, Cellulomonas* et *Streptomyces.* Les enzymes sécrétées par ces microorganismes possèdent trois types d'activités utiles dans la conversion de la cellulose en glucose et se divisent en trois groupes: les endoglucanases, qui attaquent les fibres de celluloses aléatoirement en interne, les exoglucanases qui vont attaquer les extrémités des fibres en libérant du cellobiose, et les beta-glucosidases qui vont hydrolyser ce cellobiose en glucose. D'autres classes d'enzymes telles que les hémicellulases ou la classe d'enzymes récemment découverte des polysaccharides mono-oxygénases peuvent jouer également un rôle dans l'efficacité de l'hydrolyse.

Il y a un intérêt industriel fort pour la diminution du coût de l'hydrolyse enzymatique, et cette diminution passe par l'utilisation d'une dose réduite d'enzymes et donc des cocktails d'enzymes plus efficaces. En conséquence, plusieurs demandes de brevets décrivent des enzymes naturelles aux capacités supérieures à celles de *Trichoderma reesei,* ou des variants améliorés par génie génétique. On peut citer les demandes de brevet US2010304464, WO2010066411, PCT/US2013/074030, US 2014/017737, US2013/130353, US2008/167214 et WO2013029176 concernant les exoglucanases, les demandes WO2007109441, WO2012149192 et WO2010076388 concernant les endoglucanases, les demandes WO2010029259, WO2010135836 ou WO2010022518 concernant les beta-glucosidases, ou encore les demandes WO12135659, WO12149344 concernant les polysaccharides mono-oxygénases. On peut également citer les numéro d'accession suivants concernant les exoglucanases : BBI75603, BBI74548, BBB73564, BAO94743, AUP68912, ASQ81185 ou ASQ81184.

La demande PCT/US2011/061482 décrit des polypeptides chimères possédant une activité renforçant l'activité cellulolytique, de même que les numéro d'accession AZW45974 et AZW45973.

La demande de brevet FR2782323 décrit un procédé de production in vitro de séquences polynucléotidiques recombinées.

Les enzymes hydrolysant la biomasse lignocellulosique sont classées dans le système CAZy (Cantarel, B. L., Coutinho, P. M., Rancurel, C., Bernard, T., Lombard, V., & Henrissat, B. (2009). The Carbohydrate-Active EnZymes database (CAZy): an expert resource for Glycogenomics. Nucleic acids research, 37, D233-8) sur des critères principalement structuraux. Les exoglucanases peuvent appartenir aux familles GH 6, 7, 9,48 et 74.

Pour qu'une hydrolyse de la biomasse lignocellulosique soit efficace et économiquement rentable, le mélange enzymatique doit comporter des proportions équilibrées d'enzymes ayant des activités enzymatiques diverses, entre autres, mais non exclusivement, du type exoglucanases, endoglucanases, xylanases et beta-glucosidases. A titre d'exemple, dans les mélanges natifs de *Trichoderma reesei,* on constate généralement la présence de 60-70% d'exoglucanases, 15-20% d'endoglucanases, quelques pourcentages d'hémicellulases et environ 5-10% de beta-glucosidases. Ce mélange convient pour hydrolyser la majorité des substrats prétraités (ex. type paille de blé explosée à la vapeur en conditions acides) avec des rendements acceptables. La proportion déjà importante des exoglucanases dans le mélange indique qu'il sera difficile d'augmenter la quantité de ces enzymes sans pénaliser les autres activités endoglucanases, hemicellulases et glucosidases. Le génome de *Trichoderma reesei* comporte deux exoglucanases, l'une issue de la famille 6 (CBH2, cel6a) et l'autre issue de la famille 7 (CBH1, Cel7a). Ces deux exoglucanases hydrolysent en cellobiose respectivement les extrémités non réductrices (EC3.2.1.176) et réductrices (EC3.2.1.91) de la cellulose.

L'hydrolyse et la fermentation peuvent être réalisées suivant différents schémas. Le plus courant consiste en une hydrolyse et une fermentation séparées (SHF - Separate Hydrolysis and Fermentation). Cette méthode permet d'optimiser chaque étape par le maintien des conditions optimales de réaction. Cette fermentation s'effectue de manière extemporanée, à une température comprise entre environ 28°C et environ 30°C, tandis que l'hydrolyse a lieu généralement à une température d'au moins 45°C. Cependant, en SHF, les sucres libérés en fin de réaction sont présents à très forte concentration et entraînent une inhibition des enzymes, ralentissant l'efficacité du procédé. Pour éviter ces inconvénients, un autre type de procédé peut être envisagé. En SSF (Simultaneous Saccharification and Fermentation), les deux étapes (hydrolyse et fermentation des hexoses) ont lieu de manière simultanée, empêchant l'accumulation des sucres à des concentrations inhibitrices pour les enzymes. Les coûts d'investissement sont également réduits grâce à l'utilisation d'un seul réacteur. Le taux d'hydrolyse est plus élevé suite à l'absence d'inhibition car les sucres libérés sont utilisés immédiatement pour la fermentation en éthanol. Dans cette méthode, la température du réacteur constitue nécessairement un compromis entre les températures optimales d'hydrolyse et de fermentation, typiquement entre environ 30°C et environ 35°C. Cependant, à une telle température, l'activité des enzymes cellulolytiques est diminuée de 30% environ.

La SSF permet également l'expression d'enzymes dégradant la cellulose dans l'organisme fermentant les sucres, ce qui permet de limiter, ou dans un cas extrême de supprimer le recours aux enzymes produites lors d'une étape séparée.

En conséquence, l'obtention d'enzymes maintenant une activité exoglucanase efficace aux températures optimales d'hydrolyse et de fermentation (soit entre 30°C et 50°C), tout en gardant la proportion de l'ensemble des enzymes du mélange, serait un gain significatif pour le procédé de conversion de biomasse lignocellulosique en biocarburant.

Les inventeurs ont développé un polypeptide ayant une activité exoglucanase améliorée, notamment par rapport à l'activité exoglucanase de la protéine de référence CBH1 de séquence SEQ ID NO :2. CBH1 correspond à l'exoglucanase 1 de *Trichoderma reesei.*

Dans cette optique, les déposants ont eu le grand mérite de trouver, après de nombreuses recherches, un polypeptide isolé ou purifié ayant une activité exoglucanase améliorée par rapport à l'activité exoglucanase de la protéine de référence CBH1 (SEQ ID NO :2).

La description concerne donc un polypeptide choisi dans le groupe consistant en :
i. une séquence d'acides aminés choisie parmi SEQ ID NO :4, SEQ ID NO :6, SEQ ID NO :8, SEQ ID NO :10, SEQ ID NO :12; SEQ ID NO :14 SEQ ID NO : 16, SEQ ID NO : 18 et SEQ ID NO :20 ; et
ii. une séquence d'acides aminés présentant un pourcentage de résidus identiques par rapport à l'une des séquences SEQ ID NO :6, SEQ ID NO :8, SEQ ID NO :10, SEQ ID NO :12; SEQ ID NO :14 SEQ ID NO :16, SEQ ID NO :18 ou SEQ ID NO :20 (ou pourcentage d'identité), d'au moins 70%, préférentiellement d'au moins 75%, 80%, 85%, 90%, 95%, 98% ou 99%.

L'invention est définie par les revendications.

De préférence, le polypeptide tel que décrit précédemment est caractérisé en ce que son expression dans un organisme fermentaire est au moins égale à l'expression de la protéine de référence CBH1 (SEQ ID NO :2).

Selon l'invention, le pourcentage d'identité d'une séquence donnée par rapport à SEQ ID NO :4, 6, 8, 10, 12, 14, 16, 18 ou 20 correspond au nombre de résidus identiques entre cette séquence donnée et SEQ ID NO :4, 6, 8, 10, 12, 14, 16, 18 ou 20 divisé par le nombre de résidus dans SEQ ID NO :4, 6, 8, 10, 12, 14, 16, 18 ou 20.

Le polypeptide de l'invention a une activité exoglucanase améliorée d'au moins 10%, préférentiellement d'au moins 20%, préférentiellement d'au moins 30%, encore plus préférentiellement d'au moins 40%, à une température d'environ 35°C et/ou d'environ 50°C, par rapport à l'activité exoglucanasse du polypeptide CBH1 de séquence d'acides aminés SEQ ID N°2.

L'homme du métier pourra par exemple déterminer l'augmentation ou autrement dit l'amélioration de l'activité enzymatique soit à l'aide d'un substrat comme la cellulose Avicel®, la cellulose PASC ou « Walseth », ou avec un substrat chromogénique (p-Nitrophenyl glycoside), par exemple le pNP lactoside. L'activité enzymatique sera respectivement révélée par dosage colorimétrique des sucres réducteurs ou bien du nitrophénol libérés.

Un exemple de protocole que l'homme du métier pourra utiliser pour déterminer si un polypeptide selon l'invention présente une activité enzymatique améliorée par rapport à celle de la protéine de référence CBH1 (SEQ ID NO :2), est le suivant :
- préparation d'une culture stock de *Y.lipolytica* exprimant une enzyme recombinante selon l'invention pendant toute la nuit à 28°C ;
- ensemencement d'un milieu d'expression avec un volume de culture stock permettant d'avoir une DO à 600 nm égale à 0.2 au début de la culture ;
- culture desdites cellules à 28°C pendant 96 heures ;
- centrifugation à 8000 rpm pendant 5 minutes ;
- incubation de 100 µL de surnageant avec 100 µL de tampon citrate phosphate 0.1 M pH 6 contenant 1 % de Cellodextrines (CD) réduites pendant 24 heures à 35°C et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la DO à 540 nm sur 150 µL.

Le TABLEAU 1 ci-dessous comprend les identifications des séquences nucléiques et peptidiques pour CBH1 de *T. reesei* ("sauvage"), les exoglucanases putatives de *Talaromyces stipitatus* (TS) et de *Neosartorya ficheri* (NF), ainsi que pour les polypeptides et nucléotides de l'invention.

**TABLEAU 1 : clones améliorés et gènes parents**

| **Clones** | **Acide nucléique** | **Polypeptide** |
|---|---|---|
| cbh1 (sauvage) | SEQ ID NO : 1 | SEQ ID NO :2 |
| 32F9 | SEQ ID NO :3 | SEQ ID NO :4 |
| 64C2 | SEQ ID NO :5 | SEQ ID NO :6 |
| 130G9 | SEQ ID NO :7 | SEQ ID NO :8 |
| 224C11 | SEQ ID NO :9 | SEQ ID NO : 10 |
| 225B11 | SEQ ID NO : 11 | SEQ ID NO : 12 |
| 242D11 | SEQ ID NO : 13 | SED ID NO : 14 |
| 453E8 | SEQ ID NO : 15 | SED ID NO : 16 |
| B | SEQ ID NO : 17 | SEQ ID NO : 18 |
| 91D9 | SEQ ID NO :19 | SEQ ID NO :20 |
| Gène NF | SEQ ID NO :21 | SED ID NO :22 |
| Gène TS | SEQ ID NO :23 | SED ID NO :24 |

L'invention a également pour objet un acide nucléique purifié ou isolé codant au moins un polypeptide tel que décrit précédemment, selon l'invention.

De préférence, ledit acide nucléique purifié ou isolé peut être choisi parmi les séquences suivantes: SEQ ID NO :3, SEQ ID NO :5, SEQ ID NO :7, SEQ ID NO :9, SEQ ID NO :11; SEQ ID NO :13, SEQ ID NO :15, SEQ ID NO :17, SEQ ID NO :19.

Selon l'invention, l'acide nucléique tel que décrit précédemment pourra être lié opérationnellement à un promoteur, un terminateur ou toute autre séquence nécessaire à son expression dans une cellule hôte.

L'invention porte également sur un vecteur comprenant au moins un acide nucléique tel que décrit précédemment, selon l'invention.

Selon l'invention, on entend par « vecteur » toute séquence d'ADN dans laquelle il est possible d'insérer des fragments d'acide nucléique étranger, les vecteurs permettant d'introduire de l'ADN étranger dans une cellule hôte. On peut citer de manière non-exhaustive comme vecteurs : les plasmides, les cosmides, les chromosomes artificiels de levures (YAC), les chromosomes artificiels de bactéries (BAC), les chromosomes artificiels dérivés du bactériophage P1 (PAC) ou les vecteurs dérivés de virus.

Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

L'invention a également pour objet une cellule hôte isolée comprenant soit au moins l'un des polypeptides tels que décrit précédemment, selon l'invention, soit au moins l'un des acides nucléiques tels que décrit précédemment, selon l'invention, ou soit au moins l'un des vecteurs tels que décrit précédemment, selon l'invention.

L'homme du métier pourra introduire l'un des polypeptides, l'un des acides nucléiques ou l'un des vecteurs tels que décrit précédemment dans la cellule hôte par des méthodes conventionnelles bien connues. Par exemple, on peut citer le traitement au chlorure de calcium, l'électroporation, l'utilisation d'un pistolet à particules.

Selon un mode de réalisation, l'homme du métier pourra introduire dans la cellule hôte et par des méthodes conventionnelles, plusieurs copies d'un acide nucléique codant un polypeptide ayant une activité exoglucanase améliorée selon l'invention.

Selon un mode de réalisation, la cellule hôte isolée telle que décrite précédemment selon l'invention est choisie parmi *Trichoderma, Aspergillus, Neurospora, Humicola, Myceliophthora, Chrysosporium, Penicillium, Fusarium, Thermomonospora, Bacillus, Pseudomonas, Escherichia, Clostridium, Cellulomonas, Streptomyces, Yarrowia, Pichia* et *Saccharomyces.*

Selon un mode de réalisation préféré, la cellule hôte isolée telle que décrite précédemment selon l'invention est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Myceliophthora thermopila, Chrysosporium lucknowense, Neurospora crassa, Humicola grisae, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

Selon un mode de réalisation préféré, la cellule hôte isolée telle que décrite précédemment selon l'invention est choisie parmi *Trichoderma reesei* et *Saccharomyces cerevisiae.*

L'invention a également pour objet l'utilisation d'au moins l'un quelconque des polypeptides décrits précédemment, selon l'invention, pour l'hydrolyse de la cellulose.

L'invention a également pour objet l'utilisation d'au moins l'un quelconque des polypeptides décrits précédemment, selon l'invention, pour la production de biocarburant.

Selon l'invention, le terme biocarburant peut être défini comme étant tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol.

Dans un autre mode de réalisation, le biocarburant est du biogaz.

Dans un autre mode de réalisation, le produit est une molécule intéressant l'industrie chimique, comme par exemple, un autre alcool tel que le 1,2-propane diol, le 1,3-propane diol, le 1,4-butane diol, le 2,3-butane diol, des acides organiques comme l'acide acétique, propionique, acrylique, butyrique, succinique, malique, fumarique, citrique, itaconique, ou des hydroxyacides comme l'acide glycolique, hydroxypropionique, ou lactique.

Un mode de réalisation de production d'un cocktail enzymatique utile pour l'hydrolyse de la lignocellulose est décrit ci-dessous.

Les souches de champignons filamenteux, de préférence *Trichoderma,* plus préférentiellement *T. reesei,* capables d'exprimer au moins un polypeptide selon l'invention sont cultivées en fermenteurs, en présence d'un substrat carboné, tel que le lactose ou le glucose, choisi pour la croissance du microorganisme. Dans un mode de réalisation, ce substrat carboné, selon sa nature, est introduit dans le fermenteur avant stérilisation ou est stérilisé séparément et introduit dans le fermenteur après stérilisation de ce dernier pour obtenir une concentration initiale de 20 à 35 g/L.

Une solution aqueuse contenant le substrat choisi pour la production des enzymes est ensuite ajoutée. Une composition enzymatique agissant sur la biomasse lignocellulosique produite par les champignons est enfin récupérée par filtration du milieu de culture. Dans cette composition, on retrouve notamment, la beta-glucosidase, l'endoglucanase et l'exoglucanase selon l'invention.

Dans un mode de réalisation, la solution aqueuse contenant le substrat choisi pour la production des enzymes est préparée à la concentration de 200-250 g/L. Cette solution contient en outre de préférence un substrat inducteur tel que le lactose. Cette solution aqueuse est injectée après l'épuisement du substrat carboné initial de façon à apporter une quantité optimisée, comprise entre 35 et 45 mg / g de cellules ("fed batch"). Pendant cette phase de "fed batch", la concentration résiduelle en sucre dans le milieu de culture est inférieure à 1 g / L et les enzymes agissant sur la biomasse lignocellulosique sont sécrétées par le champignon. Ces dernières peuvent être récupérées par filtration du milieu de culture.

L'invention a pour objet une composition enzymatique apte à agir sur la biomasse lignocellulosique, ladite composition enzymatique étant produite par des champignons filamenteux et comprenant au moins l'un quelconque des polypeptides décrits précédemment, selon l'invention.

Par « champignons filamenteux », on entend notamment *Trichoderma,* plus préférentiellement *T. reesei.*

Enfin, l'invention a pour objet un procédé de production de biocarburant à partir de la biomasse comprenant les étapes successives suivantes :
- on met en suspension en phase aqueuse la biomasse à hydrolyser;
- on hydrolyse la biomasse lignocellulosique en présence d'une composition enzymatique telle que décrite précédemment, selon l'invention, de manière à produire un hydrolysat contenant du glucose;
- on fermente en présence d'un organisme fermentaire le glucose de l'hydrolysat de manière à produire un moût de fermentation;
- on sépare le biocarburant du moût de fermentation.

Dans un mode de réalisation, la biomasse à hydrolyser est mise en suspension en phase aqueuse à raison de 6 à 40 % de matière sèche, de préférence 20 à 30 %. Le pH est ajusté entre 4 et 5,5, de préférence entre 4,8 et 5,2 et la température entre 40 et 60°C, de préférence entre 45 et 50°C. La réaction d'hydrolyse est démarrée par l'ajout de la composition enzymatique agissant sur la biomasse lignocellulosique; la quantité habituellement utilisée est de 10 à 30 mg de protéines excrétées par gramme de substrat prétraité ou moins. La réaction dure généralement de 15 à 48 heures. La réaction est suivie par dosage des sucres libérés, notamment le glucose. La solution de sucres est séparée de la fraction solide non hydrolysée, essentiellement constituée de lignine, par filtration ou centrifugation et ensuite traitée dans une unité de fermentation.

Après l'étape de fermentation, le biocarburant est séparé du moût de fermentation par exemple par distillation.

Un autre objet de l'invention est un procédé de production de biocarburant à partir de la biomasse, caractérisé en ce qu'il comprend les étapes successives suivantes :
- on met en suspension en phase aqueuse la biomasse à hydrolyser;
- on ajoute simultanément à la suspension une composition enzymatique agissant sur la biomasse lignocellulosique telle que définie précédemment, selon l'invention, et un organisme fermentaire et on fermente le mélange de manière à produire un moût de fermentation;
- on sépare le biocarburant du moût de fermentation.

De préférence, la composition enzymatique et l'organisme fermentaire sont ajoutés simultanément puis incubés à une température comprise entre 30°C et 35°C pour produire un moût de fermentation.

Selon ce mode de réalisation, la cellulose présente dans la biomasse est convertie en glucose, et en même temps, dans le même réacteur, l'organisme fermentaire (par exemple une levure) convertit le glucose en produit final selon un procédé de SSF (Simultaneous Saccharification and Fermentation) connu de l'homme du métier. Selon les capacités métaboliques et hydrolytiques de l'organisme fermentaire, le bon déroulement de l'opération peut nécessiter l'addition d'une quantité plus ou moins importante de mélange cellulolytique exogène.

Dans un autre mode de réalisation, l'organisme fermentaire produit le polypeptide objet de l'invention par sécrétion ou en surface de sa cellule, éventuellement conjointement à d'autres enzymes agissant sur la biomasse lignocellulosique, limitant ou supprimant ainsi le besoin en enzymes produites par le champignon filamenteux. De préférence, l'organisme fermentaire est une cellule hôte telle que décrite précédemment.

De préférence, les cellules hôtes produisant la composition enzymatique et/ou l'organisme fermentaire, sont ajoutées puis incubées à une température comprise entre 30°C et 35°C pour produire un moût de fermentation.

L'utilisation du polypeptide présentant une meilleure activité exoglucanase selon la présente invention présente ainsi l'avantage d'obtenir un meilleur rendement de production de glucose tout en employant moins d'enzyme qu'auparavant, ce qui présente également un avantage économique.

D'autres aspects, objets, avantages et caractéristiques de l'invention, seront présentés à la lecture de la description non restrictive qui suit et qui décrit des modes de réalisation préférés de l'invention donnés par le biais d'exemples et des Figures 1 à 8.

### Légendes des figures

La Figure 1 est un spectre de masse MALDI-TOF représentant les cellodextrines DP3 à DP11 utilisées pour le criblage.
La Figure 2 est une photo illustrant les halos d'hydrolyse générés par les enzymes sécrétées par les clones purs isolés lors du criblage de *T. reesei* dans des puits de boîtes de Pétri Walseth.
La Figure 3 est un gel d'électrophorèse bidimensionnelle comparant les sécrétomes des clones de *T. reesei* : souche de référence CL847; souche de référence CL847ΔCBH1 ; clones purs isolés n°6 et n°20 du variant 130G9 (SEQ ID NO :8).
La Figure 4 est un gel d'électrophorèse bidimensionnelle comparant les sécrétomes des clones de *T. reesei* : souche de référence CL847; souche de référence CL847ΔCBH1 et clone pur isolé n°24 du variant 453E8 (SEQ ID NO :16).
La Figure 5 est un graphique présentant les résultats de SHF pour le cocktail 453E8-24, issu de la souche n°24 exprimant le variant 453E8 (SEQ ID NO :16) et du cocktail de référence CL847 supplémenté en β-glucosidase.
La Figure 6 est un graphique présentant les résultats de SHF pour les cocktails 130G9-6 et 130G9-20 issus des souches n°6 et n°20 exprimant le variant 130G9 (SEQ ID NO :8) et du cocktail de référence CL847 supplémenté en β-glucosidase.
La Figure 7 est un graphique présentant les résultats de SSF pour le cocktail 453E8-24, issu de la souche n°24 exprimant le variant 453E8 (SEQ ID NO :16) et du cocktail de référence CL847 supplémenté en β-glucosidase.
La Figure 8 est un graphique présentant les résultats de SSF pour les 2 cocktails 130G9-6 et 130G9-20 issus des souches n°6 et n°20 exprimant le variant 130G9 (SEQ ID NO :8) et du cocktail de référence CL847 supplémenté en β-glucosidase.

### EXEMPLES

### EXEMPLE 1 : Préparation de cellodextrines réduites de DP 3-11.

### 1- Hydrolyse de la cellulose

Adapté de Y-H. Percival Zhang, L. R. Lynd Analytical Biochemistry 322 (2003), 225-232.)

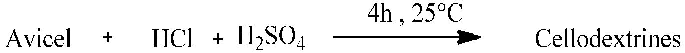

20 g de cellulose (Avicel. CAS Number 9004-34-6, Sigma-Aldrich Saint-Quentin Fallavier) sont ajoutés par portions et sous forte agitation à 160 mL d'une solution refroidie à 0°C d'acide chlorhydrique. De l'acide sulfurique, préalablement refroidi, est ajouté à la solution en plusieurs fois (4 x 10 mL). La réaction est maintenue sous agitation pendant 4 heures à 25°C avant d'être versée dans 1.8 L d'acétone refroidie à -20°C. Après 2 heures d'agitation, le précipité est filtré, repris dans 400 mL d'acétone refroidie puis filtré à nouveau. Le solide est alors repris dans 600 mL d'eau, puis agité pendant une nuit pour solubiliser les cellodextrines (CD). Après filtration du solide, la fraction soluble contenant les cellodextrines est neutralisée avec 300 g de résine Amberlite IRA 400 OH⁻, puis lyophilisée. Le lyophilisat est ensuite re-suspendu dans 500 mL de méthanol en présence d'ultra-sons pendant 30 minutes pour solubiliser les sucres de bas poids moléculaire avant d'être filtré puis lyophilisé à nouveau pour conduire à 6.8 g de cellodextrines de DP 3-11. Pour le criblage, il a été choisi de travailler avec des substrats du plus haut poids moléculaire possible pour mimer au mieux la structure de la cellulose. Mais les cellodextrines de haut poids moléculaire ne sont pas solubles, ce qui empêche une bonne reproductibilité des tests.

Une gamme de cellodextrines de DP 5-7 a donc été choisie, ce qui représente un bon compromis entre le haut poids moléculaire nécessaire et la solubilité des cellodextrines.

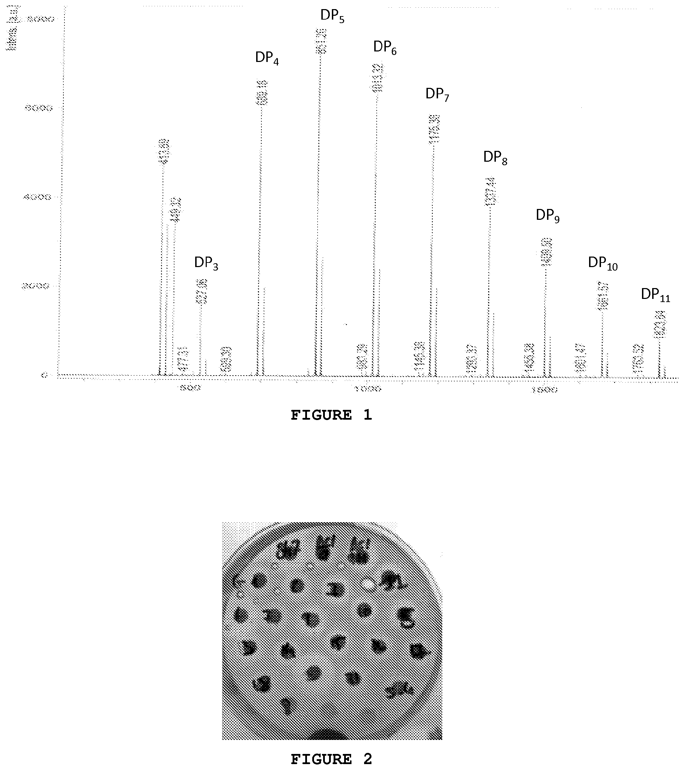

La Figure 1 présente un spectre de masse MALDI-TOF typiquement obtenu selon le procédé décrit ci-dessus.

La Figure 1 montre que les oligosaccharides isolés sont majoritairement de DP 5-7.

### 2- Réduction des cellodextrines

400 mg de borohydrure de sodium sont ajoutés à 2 g de cellodextrines DP 3-11 diluées dans 120 mL d'eau. Après 3 heures sous agitation à température ambiante, la solution est neutralisée par addition de résine Amberlite H⁺ IR 120, filtrée, puis lyophilisée, pour conduire à 2 g de cellodextrines réduites de manière quantitative. (C. Schou, G. Rasmussen, M-B. Kaltoft, B. Henrissat, M. Schulein Eur. J. Biochem. 217, 947-953 (1993)).

Un dosage au BCA (acide bicinchoninique) des cellodextrines isolées permet de vérifier la réduction totale des extrémités (Y.-H. Percival Zhang, L. R. Lynd Biomacromolecules 2005, 6, 1510-1515).

### EXEMPLE 2 : évolution par L-shuffling

La séquence du gène de la cellobiohydrolase 1 (cbhl, SEQ ID NO :1) de *Trichoderma reesei* a été soumise à un tour de L-shuffling selon le procédé breveté décrit dans le brevet EP1104457 avec les gènes d'une cellobiohydrolase de *Talaromyces stipitatus* ATCC 10500 (TS, SEQ ID NO :23) et d'une cellobiohydrolase de *Neosartorya fischeri* NRRL 181 (NF, SEQ ID NO :21) présentant respectivement 62% et 61% d'homologie avec le gène parental cbhl.

### 1-Criblase à haut débit

Un test de criblage à haut débit a été mis au point afin de sélectionner les meilleurs clones issus du L-shuffling, c'est-à-dire ceux présentant au moins 20% d'amélioration de l'activité cellobiohydrolase par rapport à l'enzyme de référence cbhl (SEQ ID NO :2).

Le test de criblage à haut débit a été réalisé selon les étapes suivantes :
- isolement sur gélose des clones de *Y. lipolytica* exprimant les variants de L-shuffling de l'enzyme selon l'invention et mise en pré-cultures en milieu YNB casa (yeast nitrogen base 1.7 g / L, NH₄Cl 10 g / L, glucose 10 g / L, casamino acids 2 g / L, pH7) desdites colonies pendant 36 heures à 28°C ;
- inoculation d'un milieu YTD (extrait de levure 10 g / L, tryptone 20 g / L, glucose 2.5 g / L, pH 6.8) additionné de tétracycline à 12.5 µg / mL à 5% avec la pré-culture puis incubation 20 heures à 28°C ;
- inoculation du milieu d'expression contenant l'inducteur (acide oléique) à raison de 20 g / L à 10% avec la culture précédente puis incubation 96 heures à 28°C ;
- centrifugation 5 minutes à 1500 rpm ;
- prélèvement de 100 µL de surnageant ;
- ajout de 100 µL de CD réduites à 1 g / L dans du tampon citrate phosphate 0.1 M à pH 6 ;
- incubation 17 heures à 50°C ;
- centrifugation pendant 5 minutes à 2500 rpm ;
- prélèvement de 80 µL de surnageant ;
- ajout de 80 µL de réactif DNS ;
- incubation 12 minutes à 105°C puis 5 minutes sur la glace ;
- lecture de la densité optique (DO) à 540 nm sur 120 µL.

Dans ces conditions de criblage, une amélioration de l'activité cellobiohydrolase (augmentation de la DO à 540 nm) par rapport à l'enzyme de référence cbhl (SEQ ID NO :2) a été trouvée dans plusieurs clones, dont notamment les clones 32F9, 64C2, 130G9, 224C11, 225B11 et 453E8 (respectivement SEQ ID NO :4, 6, 8, 10, 12 et 16).

### 2-Détermination de l'amélioration de l'activité cellobiohydrolase

### 2-1/ Sur le substrat cellodextrines réduites

Afin d'estimer le kcat relatif des variants sélectionnés au premier tour de L-shuffling par rapport à l'enzyme de référence cbhl (SEQ ID NO :2), on procède de la façon suivante :
- préparation d'une culture stock de *Y. lipolytica* exprimant une enzyme recombinante selon l'invention pendant toute la nuit à 28°C ;
- ensemencement d'un milieu d'expression avec un volume de culture stock permettant d'avoir une DO à 600 nm égale à 0.2 au début de la culture ;
- culture desdites cellules à 28°C pendant 96 heures ;
- centrifugation à 8000 rpm pendant 5 minutes ;
- incubation de 100 µL de surnageant avec 100 µL de tampon citrate phosphate 0.1 M pH 6 contenant 1 % de CD réduites pendant 24 heures à 35°C et 50°C ;
- prélèvement de 100 µL de réaction ;
- ajout de 100 µL de réactif DNS ;
- incubation 5 minutes à 100°C ;
- incubation 3 minutes sur la glace ;
- centrifugation 10 minutes à 3000 rpm ;
- lecture de la DO à 540 nm sur 150 µL.

Selon l'invention, le calcul des kcat est fait de la façon suivante :
- tracé de la courbe des DO à 540 nm en fonction de la quantité de protéine d'intérêt (en nM) ;
- soustraction de la valeur du témoin négatif ;
- division par le coefficient directeur de la gamme étalon de glucose (différentes quantités de glucose sont révélées avec le DNS) ;
- division par le temps de réaction (1440 minutes).

Le tableau 2 présente la valeur des kcat ainsi que le facteur d'amélioration obtenus pour les clones 32F9, 64C2, 130G9, 224C11, 225B11 et 453E8 (respectivement SEQ ID NO :4, 6, 8, 10, 12 et 16) par rapport à la protéine de référence cbhl (SEQ ID NO :2) dans ces conditions expérimentales.

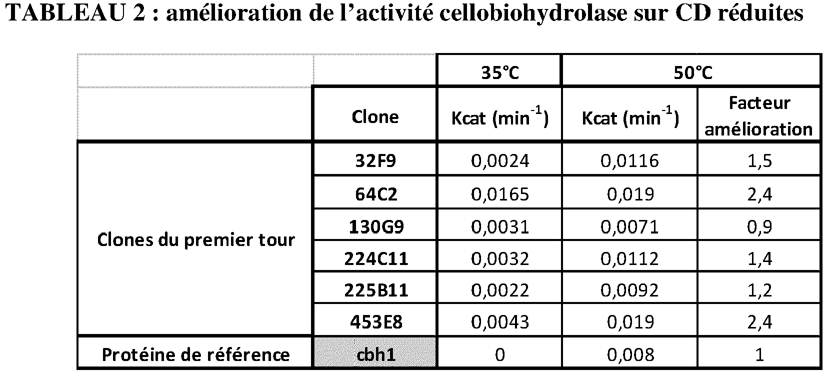

A 35°C, le facteur d'amélioration par rapport à l'enzyme de référence cbhl (SEQ ID No :2) n'a pas pu être calculé car, dans ces conditions expérimentales, l'activité de cbhl n'est pas mesurable. L'activité enzymatique des clones 32F9, 64C2, 224C11, 225B11 et 453E8 est améliorée à 35°C et 50°C par rapport à l'activité enzymatique de l'enzyme de référence cbhl (SEQ ID NO :2). L'activité enzymatique de l'enzyme 130G9 (SEQ ID No :8) est améliorée à 35°C par rapport à l'activité enzymatique de l'enzyme de référence cbhl (SEQ ID NO :2).

### 2-2/ Sur le substrat Avicel

L'amélioration d'activité des clones 32F9, 64C2, 130G9, 224C11, 225B11 et 453E8 (respectivement SEQ ID NO : 4, 6, 8, 10, 12 et 16) a ensuite été mesurée avec un second substrat : Avicel.

L'activité de ces clones a été déterminée par mesure de la DO à 540 nm en point final selon le protocole décrit précédemment. Le substrat cellodextrines réduites est remplacé par le substrat Avicel à la même concentration. Le test d'activité est réalisé avec 100 µL de surnageant de culture contenant la protéine d'intérêt pendant 48 heures.

Le tableau 3 présente la valeur des DO à 540 nm après soustraction de la valeur de DO obtenue avec le témoin négatif ainsi que le facteur d'amélioration des clones 32F9, 64C2, 130G9, 224C11, 225B11 et 453E8 (respectivement SEQ ID NO :4, 6, 8, 10, 12 et 16) par rapport à l'enzyme de référence cbhl (SEQ ID NO :2) dans ces conditions expérimentales.

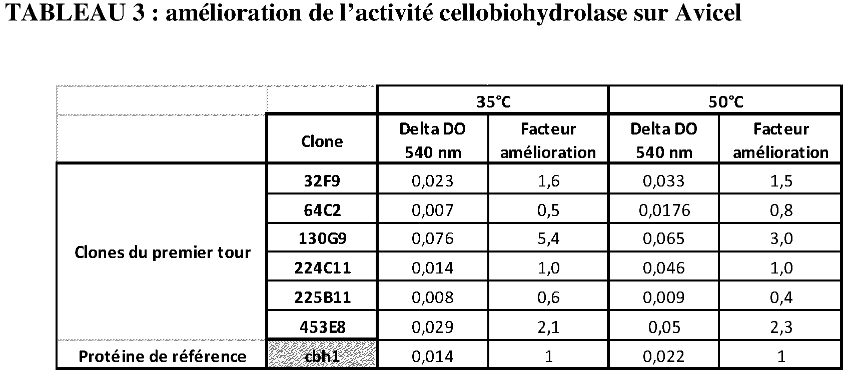

Ces résultats montrent une amélioration de l'activité enzymatique par rapport à l'enzyme de référence cbhl (SEQ ID NO :2) pour les clones 32F9, 130G9 et 453E8 (respectivement SEQ ID NO :4, SEQ ID NO :8 et SEQ ID NO :16) à 35°C et 50°C.

### EXEMPLE 3 : évolution par recombinaison

Les gènes 32F9, 130G9 et 453E8 (respectivement SEQ ID No :3, SEQ ID No :7 et SEQ ID No :15) ont été choisis car les enzymes qu'ils codent sont améliorées sur CD réduites et Avicel. Le gène 242D11 (SEQ ID No :13) a été sélectionné car sa séquence diffère de celle des clones 32F9, 130G9 et 453E8 et permet ainsi d'améliorer la diversité de séquences. Les gènes 32F9, 130G9 et 453E8 et 242D11 ont été recombinés pour générer de nouveaux mutants. L'activité des mutants obtenus a tout d'abord été évaluée avec le substrat CD réduites selon le protocole décrit au paragraphe 2-1 de l'exemple 2.

### 1-Détermination de l'amélioration de l'activité cellobiohydrolase

### 1-1/ Sur le substrat cellodextrines réduites

Le mutant B (SEQ ID No :18) a une activité cellobiohydrolase améliorée (augmentation de la DO à 540 nm) par rapport au variant 453E8 (SEQ ID NO :16). Le variant 453E8 est le meilleur variant issu de l'évolution par L-shuffling.

Le tableau 4 présente la valeur des kcat ainsi que le facteur d'amélioration obtenus pour le clone B par rapport à la protéine 453E8 (SEQ ID NO :16) dans ces conditions expérimentales. Les kcat sont calculés selon le protocole décrit au paragraphe 2-1 de l'exemple 2.

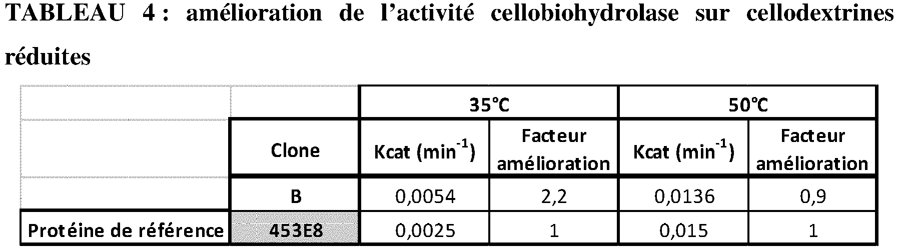

Les résultats montrent une amélioration de l'activité enzymatique par rapport à l'enzyme de référence (SEQ ID NO :16) pour le clone B (SEQ ID NO :18) à 35°C.

### 1-2/ Sur le substrat Avicel

L'amélioration d'activité du clone B a ensuite été confirmée avec un second substrat : Avicel.

L'activité de ces clones a été déterminée par mesure de la DO à 540 nm en point final selon le protocole décrit au paragraphe 2-2 de l'exemple 2.

Le tableau 5 présente la valeur des kcat ainsi que le facteur d'amélioration obtenus pour le clone B par rapport à la protéine de référence 453E8 (SEQ ID NO :16) dans ces conditions expérimentales.

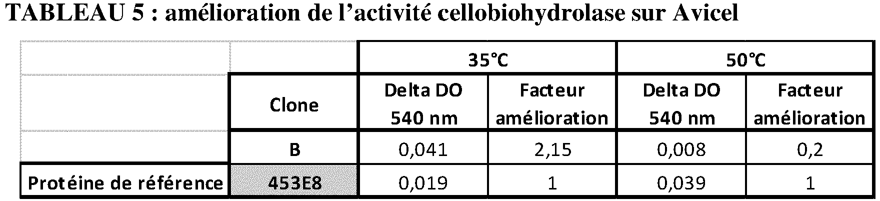

Ces résultats montrent une amélioration de l'activité enzymatique par rapport à l'enzyme 453E8 (SEQ ID NO :16) pour le clone B (SEQ ID NO :18) à 35°C.

### EXEMPLE 4 : évolution par Evosight

Afin d'améliorer l'activité cellobiohydrolase, la stratégie Evosight (demande de brevet WO2006/003298) a été appliquée au mutant 453E8 (SEQ ID No : 15), meilleur variant issu du L-shuffling.

### 1-Criblage à haut débit

Le test de criblage à haut débit utilisé pour sélectionner les meilleurs clones, c'est-à-dire ceux présentant au moins 20% d'amélioration de l'activité cellobiohydrolase par rapport à l'enzyme 453E8 (SEQ ID NO :16), est le même que celui décrit au paragraphe 1 de l'exemple 2. Les variants générés par Evosight sont comparés au clone 453E8 (SEQ ID NO :16) car il est le meilleur clone issu du L-Shuffling.

Dans ces conditions de criblage, une amélioration de l'activité cellobiohydrolase (augmentation de la DO à 540 nm) par rapport à l'enzyme 453E8 (SEQ ID NO :16) a été trouvée dans plusieurs clones, dont notamment le clone 91D9 (SEQ ID NO : 20).

### 2-Détermination de l'amélioration de l'activité cellobiohydrolase

### 2-1/ Sur le substrat cellodextrines réduites

Le protocole utilisé pour déterminer le kcat relatif du clone 91D9 (SEQ ID NO : 20) par rapport à l'enzyme 453E8 (SEQ ID NO :16) est identique à celui décrit au paragraphe 2-1 de l'exemple 1.

Le tableau 6 présente la valeur des kcat ainsi que le facteur d'amélioration obtenus pour le clone 91D9 par rapport à la l'enzyme 453E8 (SEQ ID NO :16) dans ces conditions expérimentales.

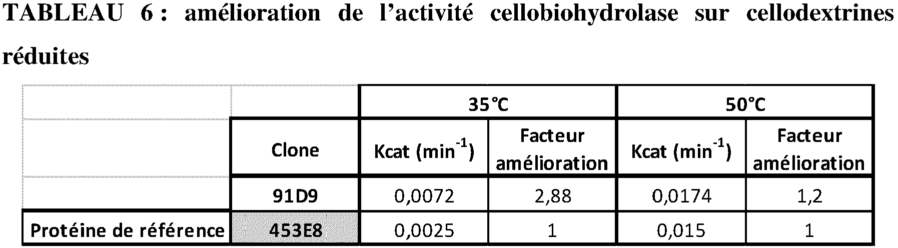

Ces résultats montrent une amélioration de l'activité enzymatique par rapport à l'enzyme 453E8 (SEQ ID NO :16) pour le clone 91D9 (SEQ ID NO :20) à 35°C et 50°C.

### 2-2/ Sur le substrat Avicel

L'amélioration d'activité du clone 91D9 a ensuite été confirmée avec un second substrat : Avicel.

L'activité de ce clone a été déterminée par mesure de la DO à 540 nm en point final selon le protocole décrit au paragraphe 2-2 de l'exemple 2.

Le tableau 7 présente la valeur du kcat ainsi que le facteur d'amélioration obtenus pour le clone 91D9 par rapport à la protéine 453E8 (SEQ ID NO :16) dans ces conditions expérimentales.

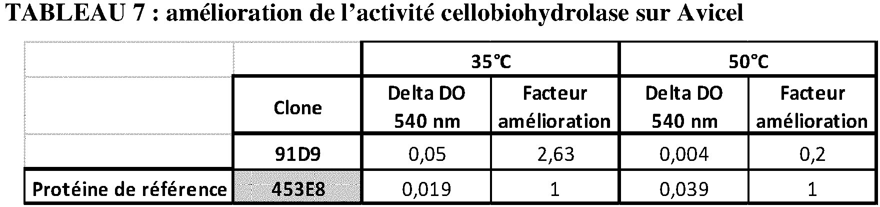

Ces résultats montrent une amélioration de l'activité enzymatique par rapport à l'enzyme 453E8 (SEQ ID NO :16) pour l'enzyme 91D9 (SEQ ID NO :20) à 35°C.

### EXEMPLE 5 : Clonage des variants 130G9 et 453E8 de l'exoglucanase 1 dans la souche T. reesei CL847 ΔCBH1

Les variants 130G9 et 453E8 sont des clones issus du L-shuffling. Chaque variant a été cloné dans une souche *T. reesei* CL847 ΔCBH1.

Les séquences codantes des variants 130G9 et 453E8 ont été amplifiées par PCR à l'aide des oligonucléotides suivants :
- **For : TCCATCctcgagatgtatcggaagttggccgtc** (SEQ ID NO :25)
- **Rev : TCCATCctcgagttacaggcactgagagtagtaag** (SEQ ID NO :26)

Les fragments obtenus ont été digérés par XhoI puis clonés dans un vecteur d'expression entre le promoteur et le terminateur *cbh1,* selon les méthodes connues de l'homme de l'art (Wang et., 2012, Microb Cell Fact. 2012 Jun 18;11:84. doi: 10.1186/1475-2859-11-84). Le marqueur de sélection du vecteur est la phléomycine (Calmels et al., 2011, Curr Genet. 1991 Sep;20(4):309-14).

La souche utilisée pour la construction est une souche CL847 (Durand et al., 1988, Enz. Microb Technol, 10, 341-346) dont le gène CBH1 a été préalablement éliminé selon une méthode connue de l'homme de l'art (Suominen et al., MGG, 1993, 241 ; 523-530) pour donner la souche CL847ΔCBH1. Des protoplastes de la souche *T*. *reesei* CL847ΔCBH1 ont été transformés selon une méthode classique connue de l'homme de l'art, par choc calcique et PEG, avec 5 µg du fragment d'ADN contenant les séquences codant les variants 130G9 ou 453E8. Les clones ainsi obtenus ont été sélectionnés sur milieu sélectif PDA / saccharose contenant 50 µg / mL de phléomycine. Le nombre de clones obtenus après purification et isolement est présenté dans le Tableau 8.

**TABLEAU 8 : Sélection des clones ayant intégré le variant d'intérêt**

| **Nom du variant** | **Nombre de clones repiqués après transformation** | **Nombre de clones purs isolés** |
|---|---|---|
| 130G9 | 231 | 19 |
| 453E8 | 189 | 11 |

L'activité des clones purs isolés est criblée sur boîtes de cellulose couplée à une analyse du sécrétome sur gel 2D.

Le milieu de criblage, dit de « Cellulose Walseth » est préparé de la façon suivante :
- 250 mL / L de milieu « 4N » (KOH 3.32 g / L, H₃PO4 85 % 5 mL / L, (NH₄)₂SO₄ 5.6 g / L, MgSO₄,7H₂O 1.2 g / L, CaCl₂,2H₂O 1.2 g / L, Na₂HPO₄,12H₂O : 0.23 g / L, pH ajusté à 1.5 avec H₂SO₄) ;
- 1 mL / L d'une solution d'oligo-éléments (FeSO₄,7H₂O 30 g / L, Co(NO₃)₂,6H₂O 9 g / L, MnSO₄,1H₂O 6.4 g / L, ZnSO₄,7H₂O 8.4 g / L, acide borique 0.4 g / L, Molybdate de Sodium 1.04 g / L, pH ajusté à 1.5 avec H₃PO₄) ;
- 2 g / L de peptone ;
- 20 g / L d'agar ;
- 50 g / L de cellulose à 8% préparée selon la méthode de Walseth (Walseth, 1952, Tappi, 225 ; 228-232).

L'ensemble est homogénéisé à l'aide d'un homogénéisateur (Ultra Turrax, Ika, Allemagne) pendant 5 minutes. Le pH est ajusté à 6.0 avec une solution de KOH 3 M. Le milieu obtenu est autoclavé à 110°C pendant 30 minutes. Lorsque la température du milieu est de 50°C, la phléomycine est ajoutée à raison de 50 µg / mL. Le milieu est ensuite transféré dans les boîtes de Pétri à raison de 20 mL / boîte. La solidification est surveillée jusqu'à la prise complète de la gélose sur laquelle est alors disposé un disque de Plexiglass perforé : 24 puits par boîte sont ainsi créés.

L'étape de criblage est réalisée en déposant des extraits de gélose portant des clones isolés à l'issue de la transformation dans les puits des boîtes de Pétri Walseth (1 clone pur isolé / puits). Ce système permet d'obtenir des halos d'hydrolyse enzymatique puisque le mycélium reste confiné dans le puits alors que les enzymes cellulolytiques sécrétées diffusent dans la gélose. Les boîtes sont incubées à 30°C durant 7 jours à l'issue desquels une évaluation visuelle des halos est réalisée par différence de couleur entre la gélose opaque et les zones hydrolysées transparentes.

La Figure 2 illustre cette technique et son pouvoir discriminant en montrant des clones d'intérêt identifiés par comparaison aux deux souches contrôles : la souche CL847ΔCBH1 (noté ΔC1 sur la boîte) et la souche CL847 non délétée du gène de référence *cbh1* (SEQ ID NO :1).

Ainsi, tout clone isolé dont le halo est inférieur à celui de CL847ΔCBH1 est écarté alors que ceux dont le halo est au moins supérieur à celui de CL847ΔCBH1 sont conservés.

En suivant cette procédure pour l'ensemble des clones obtenus, les clones n°6 et n°20 issus de la transformation de la souche CL847ΔCBH1 par la séquence codant le gène 130G9 (SEQ ID NO :7) et le clone isolé n°24 issu de la transformation de la souche CL847ΔCBH1 par la séquence codant le gène 453E8 (SEQ ID NO :15) ont ainsi été sélectionnés et conservés.

Afin de confirmer ce choix, les trois clones isolés sélectionnés ont été mis en culture durant 7 jours à 30°C et sous agitation à 150 rpm en milieu liquide de composition suivante :
3.48 g K₂HPO₄, 1.68 g (NH₄)₂SO₄, 0.12 g MgSO₄, 0.6 g Cornsteep, 1 mL de solution d'oligo-éléments (30 g / L FeSO₄,7H2O, 9 g / L Co(NO₃)₂,6H₂O, 6.4 g / L MnSO₄,1H₂O, 8.4 g / L ZnSO₄,7H₂O, 0.4 g / L acide borique, 1.04 g / L molybdate de Sodium, pH ajusté à 1.5 avec H₃PO₄), 4.64 g acide maléique, 4 g lactose, 4 g cellulose Solka Floc (Nutrafiber, USA) pour 1 L de milieu. L'ensemble est homogénéisé à l'aide d'un Ultrathurax pendant 5 minutes. Le pH est ajusté à 6.0 avec une solution de KOH 3 M. Le milieu obtenu est autoclavé à 110°C pendant 30 minutes. La phléomycine est ajoutée à raison de 50 µg / mL lorsque le milieu est à température ambiante.

Un dosage de concentration protéique du milieu extracellulaire est réalisé à l'aide d'un kit colorimétrique DC Protein Assay (BioRad, Californie, États-Unis) à partir d'une gamme étalon d'albumine de sérum bovin (BSA). Les surnageants font ensuite l'objet d'une électrophorèse bidimensionnelle telle que décrite par Herpoél-Gimbert et al. (Biotechnol Biofuels. 2008 Dec 23;1(1):18. doi: 10.1186/1754-6834-1-18), à l'aide de strips de 7 cm pH 4.0 - 7.0.

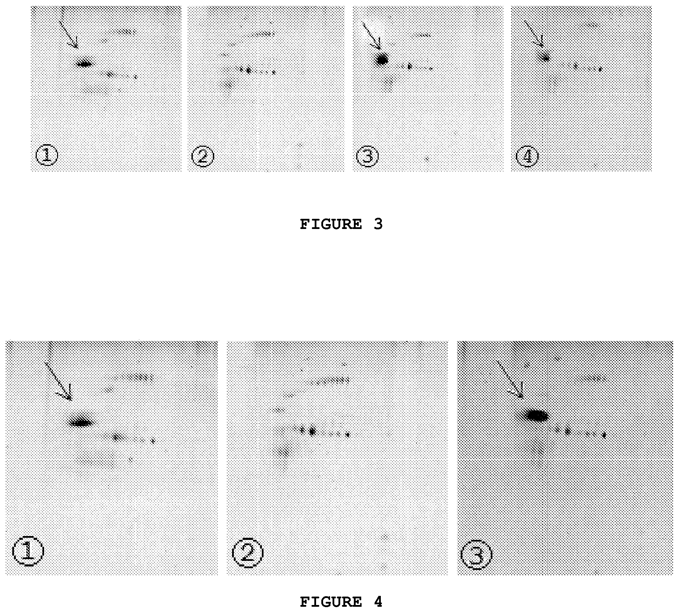

Les profils protéiques obtenus pour les clones n°6 et n°20 du variant 130G9 (SEQ ID NO :8) et pour le clone n°24 du variant 453E8 (SEQ ID NO :16) sont comparés à ceux des souches références CL847 et CL847ΔCBH1 (Figure 3 et Figure 4).

Les résultats présentés en Figure 3 et en Figure 4 montrent que l'intensité des spots, qui correspondent aux protéines du sécrétome, est similaire dans chaque souche. Ceci permet de vérifier que l'expression de ces protéines est conservée quelle que soit la souche. La bande indiquée par les flèches permet de confirmer la présence de CBH1 dans ces souches, par comparaison avec la souche ayant servi aux transformations CL847ΔCBH1.

Les clones sélectionnés à l'issue de ces étapes de criblage sont nommés « souches » dans la suite des exemples.

### Exemple 6 : Production de cocktails d'enzymes

Les souches n°6 et n°20 ayant intégré le variant 130G9 (SEQ ID NO :8) et la souche n°24 ayant intégré le variant 453E8 (SEQ ID NO :16) construites dans l'exemple 5 ont fait l'objet de productions d'enzymes selon le protocole miniaturisé décrit dans la demande de brevet FR2989385 et Jourdier et al. (Microb Cell Fact.2012 May 30;11:70. doi: 10.1186/1475-2859-11-70). L'ensemble des protéines sécrétées par une souche donnée constitue son cocktail.

La production de protéines par les souches de *T. reesei* se fait en deux phases : une première phase batch pour la production de la biomasse et une deuxième phase fed-batch pour la production de protéines.

La production est réalisée selon le protocole suivant :
- Dans des fioles de 250 mL, 55 mL de milieu F45 (10 g / L de tampon dipotassium phtalate pH 6, 4.2 g / L (NH4)2SO₄, 300 mg / L MgSO₄.7H₂O, 150 mg / L CaCl₂.2H₂O, 1,5 g / L cornsteep, 0,07% d'acide orthophosphorique, 5 mg / L FeSO₄, 1,4 mg / L MnSO₄, 1,4 mg / L ZnSO₄, 3,7 mg / L CoCl₂ et 12,5 g / L glucose) sont ensemencés avec des spores des souches respectives et agitées à 150 rpm et 30°C.
- Des prélèvements sont réalisés toutes les 24 heures pour déterminer le pH et la concentration en glucose.

Dès que la concentration en glucose est inférieure à 3 g/L, la phase fed-batch est lancée par ajout d'une solution de lactose 50 g/L et de NH₃ 0,3% à un débit de 40 mg de sucre/g de biomasse par heure. Des prélèvements journaliers sont réalisés pour déterminer le pH, le poids sec et la concentration de protéines dans le surnageant. Après 5 jours de culture en fed-batch, la culture est filtrée sur un filtre de 0,45 µm et le surnageant congelé après mesure de la concentration en protéines. Celle-ci a été mesurée par la méthode de Lowry en utilisant la BSA pour réaliser la gamme étalon.

Les concentrations en protéines des surnageants obtenus pour les souches 453E8-24, 130G9-6, 130G9-20 ainsi que la souche CL847 de référence sont présentées en Tableau 9.

**Tableau 9 : Concentration en protéines des surnageants de culture**

| **Souche** | **Concentration en protéines (g/L)** |
|---|---|
| 453E8-24 | 5,3 |
| 130G9-6 | 7,4 |
| 130G9-20 | 5,8 |
| CL847 | 5,2 |

### Exemple 7 : Efficacité des enzymes issues du L-shuffling en hydrolyse de biomasse lignocellulosique selon un procédé SHF

Le substrat de référence utilisé est une paille de blé ayant subi un prétraitement par explosion à la vapeur (19 bars - 3 minutes). La biomasse subit l'explosion après imprégnation acide à 0.01%H₂SO₄ pendant 10 heures. Elle est ensuite lavée, ajustée à pH 5, pressée et séchée. Les caractéristiques de la paille sont présentées dans le Tableau 10.

**Tableau 10 : Composition de la paille utilisée pour les essais d'hydrolyse**

| **Composition** | **% m / m** |
|---|---|
| WIS | 97,52 |
| Teneur en cendres | 5 |
| Cellulose | 51,7 |
| Xylanes corrigés | 3,57 |
| Hémicellulose | 4,14 |
| Lignine de Klason (surestimée) | 36,49 |
| Acétyl | 0,6 |

Les hydrolyses ont été réalisées à 10 % de matière sèche m / m soit un équivalent de 5.4% de cellulose m / m. Le taux de WIS (Water Insoluble Solids) est systématiquement déterminé avant chaque série de microhydrolyses. La valeur WIS de référence est 93,7 %. La teneur en matière sèche lignocellulosique des essais a été fixée à 10 % soit -5.4 % de cellulose.

Le taux de protéines est fixé à 10 mg / g MS soit environ 19 mg / g cellulose. Les cocktails enzymatiques ont été supplémentés en activité β-glucosidase à hauteur de 120 ± 2 UI / g cellulose, en ajoutant de la β-glucosidase SP188 (Novozymes, Danemark). Cet ajout de β-glucosidase permet de limiter l'inhibition des cellobiohydrolases par le cellobiose.

Les essais sont effectués en tubes Eppendorf de 2 mL utile (1 g réactionnel) contenant :
- 0,11 ± 0.001 g de substrat paille lavée ;
- 0.9 ± 0.02 mL de milieu réactionnel d'hydrolyse composé de tampon acétate 50 mM - pH 4,8 et chloramphénicol (0,05 g / L) ;
- entre 0.1 et 0.2 ± 0.02 g de cocktail enzymatique en fonction de leur taux de protéines ;

Les hydrolyses enzymatiques sont réalisées à 45 ± 2 °C sous agitation type vortex à 900 rotations par minute dans un *Thermomixer Comfort Eppendorf.*

Tous les essais sont effectués en double avec des temps de prélèvement fixés à 24, 48 et 96 heures avec pour certains des prélèvements à 72 heures.

A chaque temps de prélèvement, les hydrolysats sont ébouillantés 5 minutes dans les tubes Eppendorf sacrifiés. Ces tubes sont ensuite refroidis et centrifugés. Le dosage du glucose est effectué par HPLC. Parallèlement les résidus solides de chaque tube Eppendorf sont lavés et centrifugés 3 fois avant d'être séchés à 105°C pendant 24 heures de façon à évaluer les WIS. Le calcul du rendement d'hydrolyse est effectué en tenant compte de la quantité des WIS dans la paille utilisée dans les essais d'hydrolyse.

Les trois cocktails issus des souches recombinantes 130G9-6, 130G9-20 et 453E8-24 de l'exemple 6 ont été évalués. Un test témoin est effectué avec le cocktail de référence CL847 comportant l'enzyme CBH1 native supplémenté également en β-glucosidase pour comparaison.

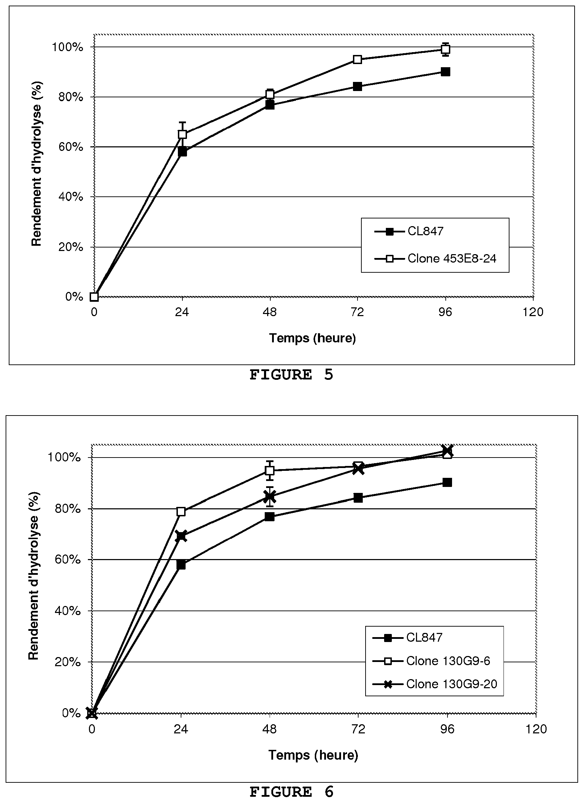

La Figure 5 présente les résultats d'hydrolyse pour le cocktail 453E8-24 comprenant l'enzyme 453E8 (SED ID NO :16).

Les résultats présentés en Figure 5 montrent que la vitesse initiale d'hydrolyse du cocktail 453E8-24 est proche de celle du cocktail de référence CL847. Le rendement final d'hydrolyse du cocktail 453E8-24 est supérieur à celui du cocktail de référence CL847.

La Figure 6 présente les résultats d'hydrolyse pour les 2 cocktails 130G9-6 et 130G9-20 issus des souches exprimant l'enzyme 130G9 (SEQ ID NO :8).

Les résultats présentés en Figure 6 montrent pour les deux cocktails 130G9-6 et 130G9-20 que la vitesse initiale d'hydrolyse est supérieure à la vitesse d'hydrolyse du cocktail de référence CL847 pendant les 20 premières heures de réaction. Le rendement final d'hydrolyse des deux cocktails 130G9-6 et 130G9-20 est également supérieur à celui du cocktail de référence CL847.

### Exemple 8 : Efficacité des enzymes en hydrolyse de biomasse lignocellulosique selon un procédé SSF

Le substrat utilisé est le même que celui décrit en tableau 10 de l'exemple 7.

Les SSF sont réalisées en triplicat dans des réacteurs de laboratoire. Ces réacteurs sont constitués des éléments suivants :
- un flacon en verre de 30 mL de volume utile ;
- un bouchon de sécurité en polyétheréthercétone (Peek) ;
- une soupape unidirectionnelle DV-118 (Vaplock, États-Unis) fixée à travers le bouchon. La soupape est configurée pour s'ouvrir en sortie lorsque la pression relative dans le flacon est supérieure à 70 mbar ;
- un premier tube creux en polypropylène dont l'extrémité inférieure est équipée d'un septum. Ce tube est emmanché à travers un second tube qui traverse le bouchon de sécurité ;
- un joint plat disposé entre le goulot du flacon et le bouchon de sécurité.

Le principe de mise en œuvre des bioréacteurs est le suivant : le CO₂ produit lors de la fermentation éthanolique s'accumule dans le ciel situé au-dessus du milieu réactionnel entrainant par accumulation une augmentation de la pression dans le bioréacteur (P_{G}). Lorsque P_{G} devient supérieure à la pression d'ouverture de la soupape unidirectionnelle (P_{S}), la soupape s'ouvre pour laisser échapper une quantité de gaz qui est par exemple déterminée par pesée. Lorsque P_{G} < P_{S}, la soupape se referme jusqu'à ce que P_{G} soit supérieur à P_{S}. Ainsi le bioréacteur en fonctionnement est toujours sous pression de manière à assurer un milieu anaérobie stable pour la fermentation. La quantité d'éthanol produite est évaluée par la production de CO₂ estimée par perte de poids à partir de l'équation stœchiométrique de fermentation du glucose en éthanol suivante :

**C₆H₁₂O₆ (glucose) → 2 CO₂ + 2 CH₃CH₂OH (éthanol) + énergie**

Le milieu de culture utilisé pour la SSF est un milieu aqueux qui comprend :
- un tampon acétate 50 mM pour pH 5 ;
- du chloramphénicol à 0,1 g / L ;
- du milieu nutritif à 3 g / L de KH₂PO₄, 2 g / L de (NH₄)₂SO₄, 0,4 g / L de MgSO₄,7H₂O et 1 g / L d'extrait de levure.

Les SSF ont été réalisées à 10 ± 0.01 % m / m de matière sèche soit un équivalent de 5.4 % cellulose m / m pour une masse réactionnelle totale de 15 ± 0.003 g. Le taux de protéines est fixé à 10 ± 0.01 mg de cellulases par gramme de matière sèche, soit environ 19 mg / g cellulose. Les cocktails enzymatiques ont été supplémentés en activité β-glucosidase à hauteur de 120 ± 2 UI / g cellulose, en ajoutant de la β-glucosidase SP188 (Novozymes, Danemark).

La levure de fermentation des sucres (*Saccharomyces cerevisiae,* souche Ethanol Red, Fermentis, France) est ajoutée dans le milieu pour obtenir une teneur de 2 ± 0.1 g / kg.

Les enzymes et la levure sont ajoutées dans les bioréacteurs après une heure de conditionnement de la paille de blé prétraitée à 35°C avec le milieu de culture.

La réaction de SSF est conduite à une température d'environ 35°C, en plaçant le bioréacteur de laboratoire dans un incubateur de type INFORS Multitron HT Standard avec une vitesse de rotation orbitale de 150 tours par minute.

Au cours du temps, on a suivi la perte de masse par pesée des bioréacteurs. A la fin de la réaction, le moût de fermentation est chauffé à 100°C pendant 5 minutes, refroidi et centrifugé pour séparer les matières solides non hydrolysées du jus de fermentation. Le jus de fermentation est ensuite analysé par chromatographie en phase gazeuse afin de déterminer sa concentration en éthanol.

Les trois cocktails issus des souches recombinantes 130G9-6, 130G9-20 et 453E8-24 de l'exemple 6 ont été évalués. Une SSF est effectuée avec le cocktail de référence comportant l'enzyme CBH1 native supplémenté également en β-glucosidase pour comparaison.

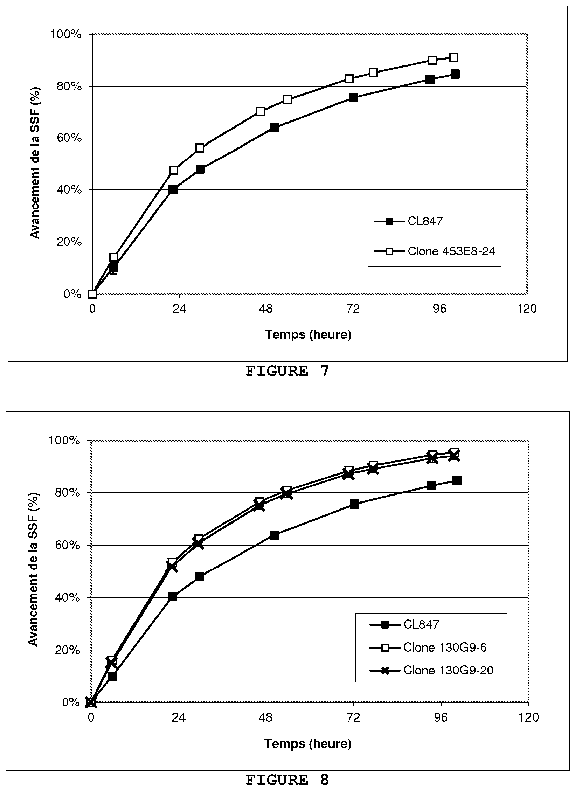

La Figure 7 présente les résultats d'avancement de la SSF pour le cocktail 453E8-24 issu des souches exprimant l'exoglucanase 453E8.

Les résultats présentés en Figure 7 montrent que la concentration en éthanol après 100 heures de SSF est équivalente dans le jus de fermentation du cocktail 453E8-24 et dans celui de la souche de référence CL847.

La Figure 8 présente les résultats d'avancement de la SSF pour les cocktails 130G9-6 et 130G9-20 issus des souches exprimant l'enzyme du clone 130G9 (SEQ ID :NO 8).

Les résultats présentés en Figure 8 montrent pour les deux cocktails 130G9-6 et 130G9-20 que la vitesse initiale de fermentation est supérieure à celle du cocktail de référence CL847 pendant les 20 premières heures de réaction. Le rendement final des deux cocktails 130G9-6 et 130G9-20 est également supérieur à celui du cocktail de référence CL847.

## Revendications

1. Polypeptide isolé ou purifié **caractérisé en ce qu'**il a une activité exoglucanase améliorée d'au moins 10% à une température d'environ 35°C, et optionnellement d'au moins 10% à une température d'environ 50°C, par rapport à l'activité exoglucanase de la protéine de référence CBH1 de séquence SEQ ID NO : 2 sur un substrat choisi parmi la cellulose Avicel, la cellulose PASC, un substrat cellodextrines réduites ou un substrat chromogénique tel que le pNP lactoside, ledit polypeptide étant choisi dans le groupe consistant en :
i. une séquence d'acides aminés choisie parmi SEQ ID NO : 8, SEQ ID NO : 4, SEQ ID NO : 16, SEQ ID NO : 6, SEQ ID NO : 10, SEQ ID NO : 12, SEQ ID NO : 18 et SEQ ID NO : 20, et
ii. une séquence d'acides aminés présentant, par rapport à la séquence SEQ ID NO : 4, SEQ ID NO : 6, SEQ ID NO : 12, SEQ ID NO : 18 ou SEQ ID NO : 20, un pourcentage d'identité d'au moins 99%.

2. Acide nucléique purifié ou isolé, **caractérisé en ce qu'**il code au moins un polypeptide selon la revendication **1.**

3. Acide nucléique purifié ou isolé selon la revendication **2** choisi parmi les séquences suivantes : SEQ ID NO : 7, SEQ ID NO : 3, SEQ ID NO : 15, SEQ ID NO : 5, SEQ ID NO : 9, SEQ ID NO : 11, SEQ ID NO : 17 et SEQ ID NO : 19.

4. Vecteur **caractérisé en ce qu'**il comprend au moins un acide nucléique selon l'une des revendications **2 ou 3.**

5. Cellule hôte isolée **caractérisée en ce qu'**elle comprend au moins un polypeptide selon la revendication **1,** ou au moins un acide nucléique selon l'une des revendications **2 ou 3,** ou au moins un vecteur selon la revendication **4.**

6. Cellule hôte isolée selon la revendication **5, caractérisée en ce qu'**elle est choisie parmi *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* et *Saccharomyces.*

7. Cellule hôte isolée selon l'une des revendications **5 ou 6, caractérisée en ce qu'**elle est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* et *Saccharomyces cerevisiae.*

8. Utilisation dudit polypeptide selon la revendication **1** pour l'hydrolyse de la cellulose.

9. Utilisation dudit polypeptide selon la revendication **1** pour la production de biocarburant.

10. Composition enzymatique apte à agir sur la biomasse lignocellulosique, ladite composition enzymatique étant produite par des champignons filamenteux et comprenant au moins un polypeptide selon la revendication **1.**

11. Procédé de production de biocarburant à partir de biomasse lignocellulosique, **caractérisé en ce qu'**il comprend les étapes successives suivantes :
- on met en suspension en phase aqueuse la biomasse à hydrolyser ;
- on hydrolyse en présence d'une composition enzymatique selon la revendication **10** la biomasse lignocellulosique de manière à produire un hydrolysat contenant du glucose ;
- on fermente le glucose de l'hydrolysat de manière à produire un moût de fermentation ;
- on sépare le biocarburant du moût de fermentation.

12. Procédé de production de biocarburant à partir de la biomasse, **caractérisé en ce qu'**il comprend les étapes successives suivantes :
- on met en suspension en phase aqueuse la biomasse à hydrolyser ;
- on ajoute simultanément d'une composition enzymatique selon la revendication **10** et un organisme fermentaire dans la suspension et on fermente le mélange de manière à produire un moût de fermentation ;
- on sépare le biocarburant du moût de fermentation.

13. Procédé selon l'une des revendications **11 ou 12,** dans lequel l'organisme fermentaire est une cellule hôte selon l'une des revendications **5 à 7.**

## Patentansprüche

1. Isoliertes oder gereinigtes Polypeptid, **dadurch gekennzeichnet, dass** es eine verbesserte Exoglucanase-Aktivität von mindestens 10% bei einer Temperatur von etwa 35°C aufweist und gegebenenfalls von mindestens 10% bei einer Temperatur von etwa 50°C im Vergleich zur Exoglucanase-Aktivität des Referenzproteins CBH1 der Sequenz SEQ ID NO : 2, auf einem Substrat, ausgewählt aus Avicel-Cellulose, PASC-Cellulose, einem reduzierten Cellodextrin-Substrat oder einem chromogenen Substrat, wie pNP-Lactosid, wobei das Polypeptid ausgewählt ist aus der Gruppe bestehend aus:
i. einer Aminosäuresequenz, ausgewählt aus SEQ ID NO:8, SEQ ID NO:4, SEQ ID NO:16, SEQ ID NO:6, SEQ ID NO:10, SEQ ID NO:12, SEQ ID NO:18, und SEQ ID NO:20; und
ii. einer Aminosäuresequenz, die in Bezug auf die Sequenz SEQ ID NO: 4, SEQ ID NO: 6, SEQ ID NO: 12; SEQ ID NO: 18 oder SEQ ID NO: 20 eine prozentuale Identität von mindestens 99 % aufweist.

2. Gereinigte oder isolierte Nukleinsäure, **dadurch gekennzeichnet, dass** sie mindestens ein Polypeptid nach Anspruch 1 kodiert.

3. Gereinigte oder isolierte Nukleinsäure nach Anspruch 2, ausgewählt aus den folgenden Sequenzen: SEQ ID NO: 7, SEQ ID NO :3, SEQ ID NO :15, SEQ ID NO :5, SEQ ID NO :9, SEQ ID NO :11; SEQ ID NO :17 und SEQ ID NO:19.

4. Vektor, **dadurch gekennzeichnet, dass** er wenigstens eine Nukleinsäure nach einem der Ansprüche 2 oder 3 umfasst.

5. Isolierte Wirtszelle, **dadurch gekennzeichnet, dass** sie wenigstens ein Polypeptid nach Anspruch 1 oder wenigstens eine Nukleinsäure nach einem der Ansprüche 2 oder 3 oder wenigstens einen Vektor nach Anspruch 4 umfasst.

6. Isolierte Wirtszelle nach Anspruch 5, **dadurch gekennzeichnet, dass** sie ausgewählt ist aus *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* und *Saccharomyces.*

7. Isolierte Wirtszelle nach einem der Ansprüche 5 oder 6, **dadurch gekennzeichnet, dass** sie ausgewählt ist aus *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermopila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* und *Saccharomyces cerevisiae.*

8. Verwendung des Polypeptids nach Anspruch 1 für die Hydrolyse von Zellulose.

9. Verwendung des Polypeptids nach Anspruch 1 für die Herstellung von Biokraftstoff.

10. Enzymzusammensetzung, die in der Lage ist, auf lignozellulosehaltige Biomasse einzuwirken, wobei die Enzymzusammensetzung durch filamentöse Pilze hergestellt wird und mindestens ein Polypeptid nach Anspruch 1 umfasst.

11. Verfahren zur Herstellung von Biokraftstoff aus lignozellulosehaltiger Biomasse, **dadurch gekennzeichnet, dass** es die folgenden aufeinanderfolgenden Schritte umfasst:
- Suspendieren der zu hydrolysierenden Biomasse in wässriger Phase;
- Hydrolysieren der lignozellulosehaltigen Biomasse in Gegenwart einer enzymatischen Zusammensetzung nach Anspruch 10, um ein Hydrolysat zu erzeugen, das Glucose enthält;
- Fermentieren der Glucose aus dem Hydrolysat, um einen Fermentationsmost zu erzeugen;
- Abtrennen des Biokraftstoffs von dem Fermentationsmost.

12. Verfahren zur Herstellung von Biokraftstoff aus Biomasse, **dadurch gekennzeichnet, dass** es die folgenden aufeinanderfolgenden Schritte umfasst:
- Suspendieren der zu hydrolysierenden Biomasse in wässriger Phase;
- gleichzeitiges Zugeben einer Enzymzusammensetzung nach Anspruch 10 und eines fermentierenden Organismus zu der Suspension und Fermentieren der Mischung, um einen Fermentationsmost zu erzeugen;
- Abtrennen des Biokraftstoffs von dem Fermentationsmost.

13. Verfahren nach einem der Ansprüche 11 oder 12, worin der fermentierende Organismus eine Wirtszelle nach einem der Ansprüche 5 bis 7 ist.

## Claims

1. Isolated or purified polypeptide **characterised in that** it has an improved exoglucanase activity by at least 10% at a temperature of about 35°C, and optionally by at least 10% at a temperature of about 50°C, with respect to the exoglucanase activity of the reference protein CBH1 of sequence SEQ ID NO: 2 on a substrate selected from Avicel cellulose, PASC cellulose, a reduced cellodextrin substrate or a chromogenic substrate such as pNP lactoside, said polypeptide being selected from the group consisting of:
i. an amino acid sequence selected from SEQ ID NO: 8, SEQ ID NO: 4, SEQ ID NO: 16, SEQ ID NO: 6, SEQ ID NO: 10, SEQ ID NO: 12, SEQ ID NO: 18 and SEQ ID NO: 20, and
ii. an amino acid sequence having, with respect to the sequence SEQ ID NO: 4, SEQ ID NO: 6, SEQ ID NO: 12, SEQ ID NO: 18 or SEQ ID NO: 20, a percentage identity of at least 99%.

2. Purified or isolated nucleic acid, **characterised in that** it encodes at least one polypeptide according to claim 1.

3. Purified or isolated nucleic acid according to claim 2 selected from the following sequences: SEQ ID NO: 7, SEQ ID NO: 3, SEQ ID NO: 15, SEQ ID NO: 5, SEQ ID NO: 9, SEQ ID NO: 11, SEQ ID NO: 17 and SEQ ID NO: 19.

4. Vector **characterised in that** it comprises at least one nucleic acid according to one of claims 2 or 3.

5. Isolated host cell **characterised in that** it comprises at least one polypeptide according to claim 1, or at least one nucleic acid according to one of claims 2 or 3, or at least one vector according to claim 4.

6. Isolated host cell according to claim 5, **characterised in that** it is selected from *Trichoderma, Aspergillus, Neurospora, Humicola, Penicillium, Fusarium, Thermomonospora, Myceliophthora, Chrysosporium, Bacillus, Pseudomonas, Escherichia, Clostridium, Cellulomonas, Streptomyces, Yarrowia, Pichia* and *Saccharomyces.*

7. Isolated host cell according to one of claims 5 or 6, **characterised in that** it is selected from *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Myceliophthora thermophila, Chrysosporium lucknowense, Penicillium pinophilum, Penicillium oxalicum, Escherichia coli, Clostridium acetobutylicum, Clostridium saccharolyticum, Clostridium benjerinckii, Clostridium butylicum, Pichia pastoris, Yarrowia lipolityca* and *Saccharomyces cerevisiae.*

8. Use of said polypeptide according to claim 1 for cellulose hydrolysis.

9. Use of said polypeptide according to claim 1 for biofuel production.

10. Enzyme composition capable of acting upon lignocellulosic biomass, said enzyme composition being produced by filamentous fungi and comprising at least one polypeptide according to claim 1.

11. Method for producing biofuel from lignocellulosic biomass, **characterised in that** it comprises the following successive steps:
- suspending the biomass to be hydrolysed in aqueous phase;
- hydrolysing in the presence of an enzyme composition according to claim 10 lignocellulosic biomass so as to produce a hydrolysate containing glucose;
- fermenting the glucose of the hydrolysate so as to produce a fermentation wort;
- separating the biofuel from the fermentation wort.

12. Method for producing biofuel from lignocellulosic biomass, **characterised in that** it comprises the following successive steps:
- suspending the biomass to be hydrolysed in aqueous phase;
- simultaneously adding an enzyme composition according to claim 10 and a fermentative organism into the suspension and fermenting the mixture so as to produce a fermentation wort;
- separating the biofuel from the fermentation wort.

13. Method according to one of claims 11 or 12, wherein the fermentative organism is a host cell according to one of claims 5 to 7.


### SEQUENCE LISTING

<110> IFP Energies Nouvelles et al
<120> VARIANTS D'EXOGLUCANASES A ACTIVITE AMELIOREE ET LEURS UTILISATIONS
<130> BFF140337
<160> 26
<170> PatentIn version 3.5
<210> 1
   <211> 1545
   <212> DNA
   <213> Trichoderma reesei
<400> 1
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg cctacgcgtc cacgtacgga     300
   gttaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctctacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cacgactgtc ggccaggaga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc    1020
   aacgagctca acgatgatta ctgcacagct gaggaggcag aattcggcgg atcctctttc    1080
   tcagacaagg gcggcctgac tcagttcaag aaggctacct ctggcggcat ggttctggtc    1140
   atgagtctgt gggatgatta ctacgccaac atgctgtggc tggactccac ctacccgaca    1200
   aacgagacct cctccacacc cggtgccgtg cgcggaagct gctccaccag ctccggtgtc    1260
   cctgctcagg tcgaatctca gtctcccaac gccaaggtca ccttctccaa catcaagttc    1320
   ggacccattg gcagcaccgg caaccctagc ggcggcaacc ctcccggcgg aaacccgcct    1380
   ggcaccacca ccacccgccg cccagccact accactggaa gctctcccgg acctacccag    1440
   tctcactacg gccagtgcgg cggtattggc tacagcggcc ccacggtctg cgccagcggc    1500
   acaacttgcc aggtcctgaa cccttactac tctcagtgcc tgtaa                    1545
<210> 2
   <211> 514
   <212> PRT
   <213> Trichoderma reesei
<400> 2
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Ala Tyr Ala
                   85                  90                  95
   Ser Thr Tyr Gly Val Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Glu Thr Ser Ser Thr Pro Gly Ala Val Arg Gly Ser Cys Ser Thr
                   405                 410                 415
   Ser Ser Gly Val Pro Ala Gln Val Glu Ser Gln Ser Pro Asn Ala Lys
               420                 425                 430
   Val Thr Phe Ser Asn Ile Lys Phe Gly Pro Ile Gly Ser Thr Gly Asn
           435                 440                 445
   Pro Ser Gly Gly Asn Pro Pro Gly Gly Asn Pro Pro Gly Thr Thr Thr
       450                 455                 460
   Thr Arg Arg Pro Ala Thr Thr Thr Gly Ser Ser Pro Gly Pro Thr Gln
   465                 470                 475                 480
   Ser His Tyr Gly Gln Cys Gly Gly Ile Gly Tyr Ser Gly Pro Thr Val
                   485                 490                 495
   Cys Ala Ser Gly Thr Thr Cys Gln Val Leu Asn Pro Tyr Tyr Ser Gln
               500                 505                 510
   Cys Leu
<210> 3
   <211> 1542
   <212> DNA
   <213> Artificial Sequence
<220>
   <223> Clone 32F9
<400> 3
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg cctacgcgtc cacgtacgga     300
   gttaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctttacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cacgactgtc ggccaggaga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc 1020
   aacgagctca acgatgatta ctgcacagct gaggaggcag aattcggcgg atcctctttc 1080
   tcagacaagg gcggcctgac tcagttcaag aaggctacct ctggcggcat ggttctggtc 1140
   atgagtctgt gggatgatta ctccgtcaac atgctgtggc tggactccac ctacccgaca 1200
   aacgccaccg gtacacccgg tgccgctcgc ggaacctgct ccaccagctc cggttcacct 1260
   aagaccgtcg aagccaactc tcccaacgcc aaggtcacct tctccaacat caagttcgga 1320
   cccattggca gcaccggcaa ccctagcggc ggcaaccctc ccggcggaaa cccgcctggc 1380
   accaccacca cccgccgccc agccactacc actggaagct ctcccggacc tacccagtct 1440
   cactacggcc agtgcggcgg tattggctac agcggcccca cggtctgcgc cagcggcaca 1500
   acttgccagg tcctgaaccc ttactactct cagtgcctgt aa 1542
<210> 4
   <211> 513
   <212> PRT
   <213> Artificial Sequence
<220>
   <223> Clone 32F9
<400> 4
   Met Tyr Arg Lys Leu Ala Val Ile Ser Ala Phe Leu Ala Thr Ala Arg
   1 5 10 15
   Ala Gln Ser Ala Cys Thr Leu Gln Ser Glu Thr His Pro Pro Leu Thr
   20 25 30
   Trp Gln Lys Cys Ser Ser Gly Gly Thr Cys Thr Gln Gln Thr Gly Ser
   35 40 45
   Val Val Ile Asp Ala Asn Trp Arg Trp Thr His Ala Thr Asn Ser Ser
   50 55 60
   Thr Asn Cys Tyr Asp Gly Asn Thr Trp Ser Ser Thr Leu Cys Pro Asp
   65 70 75 80
   Asn Glu Thr Cys Ala Lys Asn Cys Cys Leu Asp Gly Ala Ala Tyr Ala
   85 90 95
   Ser Thr Tyr Gly Val Thr Thr Ser Gly Asn Ser Leu Ser Ile Gly Phe
   100 105 110
   Val Thr Gln Ser Ala Gln Lys Asn Val Gly Ala Arg Leu Tyr Leu Met
   115 120 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Ser Val Asn Met Leu Trp Leu Asp Ser Thr Tyr Pro Thr
   385                 390                 395                 400
   Asn Ala Thr Gly Thr Pro Gly Ala Ala Arg Gly Thr Cys Ser Thr Ser
                   405                 410                 415
   Ser Gly Ser Pro Lys Thr Val Glu Ala Asn Ser Pro Asn Ala Lys Val
               420                 425                 430
   Thr Phe Ser Asn Ile Lys Phe Gly Pro Ile Gly Ser Thr Gly Asn Pro
           435                 440                 445
   Ser Gly Gly Asn Pro Pro Gly Gly Asn Pro Pro Gly Thr Thr Thr Thr
       450                 455                 460
   Arg Arg Pro Ala Thr Thr Thr Gly Ser Ser Pro Gly Pro Thr Gln Ser
   465                 470                 475                 480
   His Tyr Gly Gln Cys Gly Gly Ile Gly Tyr Ser Gly Pro Thr Val Cys
                   485                 490                 495
   Ala Ser Gly Thr Thr Cys Gln Val Leu Asn Pro Tyr Tyr Ser Gln Cys
               500                 505                 510
   Leu
<210> 5
   <211> 1545
   <212> DNA
   <213> Artificial Sequence
<220>
   <223> Clone 64C2
<400> 5
   atgtatcgga agttggccgt catctcggcc ttcttggcca cagctcgtgc tcagcaggtc      60
   ggtacttccc aagcggaggt gcacccgtcc atgacatggc agagctgcac tgctggtggc     120
   agctgcacta ccaacaacgg caaggtggtc atcgacgcca actggcgctg ggtgcacaaa     180
   gtaggcgact acacgaactg ctacaccggc aacacttggg acaagaccct atgtcctgac     240
   gatgcaacct gcgcgtccaa ctgcgccctg gagggtgcca actaccagtc cacgtacgga     300
   gcgaccacga gcggtgacag cctccgcctc aactttgtca ccacctctca gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctttacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cacgactgtc ggccaggaga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc    1020
   aacgagctca acgatgatta ctgcacagct gaggaggcag aattcggcgg atcctctttc    1080
   tcagacaagg gcggcctgac tcagttcaag aaggctacct ctggcggcat ggttctggtc    1140
   atgagtctgt gggatgatta ctacgccaac atgctgtggc tggactccac ctacccgaca    1200
   aacgagacct cctccacacc cggtgccgtg cgcggaagct gctccaccag ctccggtgtc    1260
   cctgctcagg tcgaatctca gtctcccaac gccaaggtca ccttctccaa catcaagttc    1320
   ggacccattg gcagcaccgg caaccctagc ggcggcaacc ctcccggcgg aaacccgcct    1380
   ggcaccacca ccacccgccg cccagccact accactggaa gctctcccgg acctacccag    1440
   tctcactacg gccagtgcgg cggtattggc tacagcggcc ccacggtctg cgccagcggc    1500
   acaacttgcc aggtcctgaa cccttactac tctcagtgcc tgtaa                    1545
<210> 6
   <211> 514
   <212> PRT
   <213> Artificial Sequence
<220>
   <223> Clone 64C2
<400> 6
   Met Tyr Arg Lys Leu Ala Val Ile Ser Ala Phe Leu Ala Thr Ala Arg
   1               5                   10                  15
   Ala Gln Gln Val Gly Thr Ser Gln Ala Glu Val His Pro Ser Met Thr
               20                  25                  30
   Trp Gln Ser Cys Thr Ala Gly Gly Ser Cys Thr Thr Asn Asn Gly Lys
           35                  40                  45
   Val Val Ile Asp Ala Asn Trp Arg Trp Val His Lys Val Gly Asp Tyr
       50                  55                  60
   Thr Asn Cys Tyr Thr Gly Asn Thr Trp Asp Lys Thr Leu Cys Pro Asp
   65                  70                  75                  80
   Asp Ala Thr Cys Ala Ser Asn Cys Ala Leu Glu Gly Ala Asn Tyr Gln
                   85                  90                  95
   Ser Thr Tyr Gly Ala Thr Thr Ser Gly Asp Ser Leu Arg Leu Asn Phe
               100                 105                 110
   Val Thr Thr Ser Gln Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Glu Thr Ser Ser Thr Pro Gly Ala Val Arg Gly Ser Cys Ser Thr
                   405                 410                 415
   Ser Ser Gly Val Pro Ala Gln Val Glu Ser Gln Ser Pro Asn Ala Lys
               420                 425                 430
   Val Thr Phe Ser Asn Ile Lys Phe Gly Pro Ile Gly Ser Thr Gly Asn
           435                 440                 445
   Pro Ser Gly Gly Asn Pro Pro Gly Gly Asn Pro Pro Gly Thr Thr Thr
       450                 455                 460
   Thr Arg Arg Pro Ala Thr Thr Thr Gly Ser Ser Pro Gly Pro Thr Gln
   465                 470                 475                 480
   Ser His Tyr Gly Gln Cys Gly Gly Ile Gly Tyr Ser Gly Pro Thr Val
                   485                 490                 495
   Cys Ala Ser Gly Thr Thr Cys Gln Val Leu Asn Pro Tyr Tyr Ser Gln
               500                 505                 510
   Cys Leu
<210> 7
   <211> 1545
   <212> DNA
   <213> Artificial Sequence
<220>
   <223> Clone 130G9
<400> 7
   atgtatcgga agttggccgt catctcggcc ttcttggcca cagctcgtgc tcagtcggcc 60
   tgcactctcc aatcggagac tcacccgcct ctgacatggc agaaatgctc gtctggtggc 120
   acgtgcactc aacagacagg ctccgtggtc atcgacgcca actggcgctg gactcacgct 180
   acgaacagca gcacgaactg ctacgatggc aacacttgga gctcgaccct atgtcctgac 240
   aacgagacct gcgcgaagaa ctgctgtctg gacggtgccg cctacgcgtc cacgtacgga 300
   gttaccacga gcggtaacag cctctccatt ggctttgtca cccagtctgc gcagaagaac 360
   gttggcgctc gcctttacct tatggcgagc gacacgacct accaggaatt caccctgctt 420
   ggcaacgagt tctctttcga tgttgatgtt tcgcagctgc cgtgcggctt gaacggagct 480
   ctttacttcg tgtccatgga cgcggatggt ggcgtgagca agtatcccac caacaccgct 540
   ggcgccaagt acggcacggg gtactgtgac agccagtgtc cccgcgatct gaagttcatc 600
   aatggccagg ccaacgttga gggctggcag ccgtcatcca acaacgcgaa cacgggcatt 660
   ggaggacacg gaagctgctg ctctgagatg gatatctggg aggccaactc catctccgag 720
   gctcttaccc cccacccttg cgacactccc ggccaggtga tctgcgaggg tgatgggtgc 780
   ggcggaactt actccgataa cagatatggc ggcacttgcg atcccgatgg ctgcgactgg 840
   aacccatacc gcctgggcaa caccagcttc tacggccctg gctcaagctt taccctcgat 900
   accaccaaga aattgaccgt tgtcacccag ttcgagacgt cgggtgccat caaccgatac 960
   tatgtccaga atggcgtcac tttccagcag cccaacgccg agcttggtag ttactctggc 1020
   aacgagctca acgatgatta ctgcacagct gaggaggcag aattcggcgg atcctctttc 1080
   tcagacaagg gcggcctgac tcagttcaag aaggctacct ctggcggcat ggttctggtc 1140
   atgagtctgt gggatgatta ctacgccaac atgctgtggc tggactccac ctacccgaca 1200
   aacgagacct cctccacacc cggtgccgtg cgcggaagct gctccaccag ctccggtgtc 1260
   cctgctcagg tcgaatctca gtctcccaac gccaaggtca ccttctccaa catcaagttc 1320
   ggacccattg gcagcaccgg caaccctagc ggcggcaacc ctcccggcgg aaacccgcct 1380
   ggcaccacca ccacccgccg cccagccact accactggaa gctctcccgg acctacccag 1440
   tctcactacg gccagtgcgg cggtattggc tacagcggcc ccacggtctg cgccagcggc 1500
   acaacttgcc aggtcctgaa cccttactac tctcagtgcc tgtaa 1545
<210> 8
   <211> 514
   <212> PRT
   <213> Artificial Sequence
<220>
   <223> Clone 130G9
<400> 8
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Ala Tyr Ala
                   85                  90                  95
   Ser Thr Tyr Gly Val Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Gln Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Asp Thr Pro Gly Gln Val Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Glu Thr Ser Ser Thr Pro Gly Ala Val Arg Gly Ser Cys Ser Thr
                   405                 410                 415
   Ser Ser Gly Val Pro Ala Gln Val Glu Ser Gln Ser Pro Asn Ala Lys
               420                 425                 430
   Val Thr Phe Ser Asn Ile Lys Phe Gly Pro Ile Gly Ser Thr Gly Asn
           435                 440                 445
   Pro Ser Gly Gly Asn Pro Pro Gly Gly Asn Pro Pro Gly Thr Thr Thr
       450                 455                 460
   Thr Arg Arg Pro Ala Thr Thr Thr Gly Ser Ser Pro Gly Pro Thr Gln
   465                 470                 475                 480
   Ser His Tyr Gly Gln Cys Gly Gly Ile Gly Tyr Ser Gly Pro Thr Val
                   485                 490                 495
   Cys Ala Ser Gly Thr Thr Cys Gln Val Leu Asn Pro Tyr Tyr Ser Gln
               500                 505                 510
   Cys Leu
<210> 9
   <211> 1542
   <212> DNA
   <213> Artificial Sequence
<220>
   <223> Clone 224C11
<400> 9
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg cctacgcgtc cacgtacgga     300
   gttaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctttacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cacgactgtc ggccaggaga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc    1020
   aacgagctca acgatgatta ctgcacagct gaggaggcag aattcggcgg atcctctttc    1080
   tcagacaagg gcggcctgac tcagttcaag aaggctacct ctggcggcat ggttctggtc    1140
   atgagtctgt gggatgatta ctacgccaac atgctgtggc tggactccac ctacccgaca    1200
   aacgccaccg gtacacccgg tgccgtgcgc ggaagctgct ccaccagctc cggtgtccct    1260
   gctcaggtcg aatctcagtc tcccaacgcc aaggtcacct tctccaacat caagttcgga    1320
   cccattggca gcaccggcaa ccctagcggc ggcaaccctc ccggcggaaa cccgcctggc    1380
   accaccacca cccgccgccc agccactacc actggaagct ctcccggacc tacccagtct    1440
   cactacggcc agtgcggcgg tattggctac agcggcccca cggtctgcgc cagcggcaca    1500
   acttgccagg tcctgaaccc ttactactct cagtgcctgt aa                       1542
<210> 10
   <211> 513
   <212> PRT
   <213> Artificial Sequence
<220>
   <223> Clone 224C11
<400> 10
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Ala Tyr Ala
                   85                  90                  95
   Ser Thr Tyr Gly Val Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Ala Thr Gly Thr Pro Gly Ala Val Arg Gly Ser Cys Ser Thr Ser
                   405                 410                 415
   Ser Gly Val Pro Ala Gln Val Glu Ser Gln Ser Pro Asn Ala Lys Val
               420                 425                 430
   Thr Phe Ser Asn Ile Lys Phe Gly Pro Ile Gly Ser Thr Gly Asn Pro
           435                 440                 445
   Ser Gly Gly Asn Pro Pro Gly Gly Asn Pro Pro Gly Thr Thr Thr Thr
   450 455 460
   Arg Arg Pro Ala Thr Thr Thr Gly Ser Ser Pro Gly Pro Thr Gln Ser
   465 470 475 480
   His Tyr Gly Gln Cys Gly Gly Ile Gly Tyr Ser Gly Pro Thr Val Cys
   485 490 495
   Ala Ser Gly Thr Thr Cys Gln Val Leu Asn Pro Tyr Tyr Ser Gln Cys
   500 505 510
   Leu
<210> 11
   <211> 1542
   <212> DNA
   <213> Artificial Sequence
<220>
   <223> Clone 225B11
<400> 11
   atgtatcgga agttggccgt catctcggcc ttcttggcca cagctcgtgc tcagtcggcc 60
   tgcactctcc aatcggagac tcacccgcct ctgacatggc agaaatgctc gtctggtggc 120
   acgtgcactc aacagacagg ctccgtggtc atcgacgcca actggcgctg gactcacgct 180
   acgaacagca gcacgaactg ctacgatggc aacacttgga gctcgaccct atgtcctgac 240
   aacgagacct gcgcgaagaa ctgctgtctg gacggtgccg cctacgcgtc cacgtacgga 300
   gttaccacga gcggtaacag cctctccatt ggctttgtca cccagtctgc gcagaagaac 360
   gttggcgctc gcctttacct tatggcgagc gacacgacct accaggaatt caccctgctt 420
   ggcaacgagt tctctttcga tgttgatgtt tcgcagctgc cgtgcggctt gaacggagct 480
   ctttacttcg tgtccatgga cgcggatggt ggcgtgagca agtatcccac caacaccgct 540
   ggcgccaagt acggcacggg gtactgtgac agccagtgtc cccgcgatct gaagttcatc 600
   aatggccagg ccaacgttga gggctgggag ccgtcatcca acaacgcgaa cacgggcatt 660
   ggaggacacg gaagctgctg ctctgagatg gatatctggg aggccaactc catctccgag 720
   gctcttaccc cccacccttg cacgactgtc ggccaggaga tctgcgaggg tgatgggtgc 780
   ggcggaactt actccgataa cagatatggc ggcacttgcg atcccgatgg ctgcgactgg 840
   aacccatacc gcctgggcaa caccagcttc tacggccctg gctcaagctt taccctcgat 900
   accaccaaga aattgaccgt tgtcacccag ttcgagacgt cgggtgccat caaccgatac 960
   tatgtccaga atggcgtcac tttccagcag cccaacgccg agcttggtag ttactctggc 1020
   aacgagctca acgatgatta ctgcacagct gaggaggcag aattcggcgg atcctctttc 1080
   tcagacaagg gcggcctgac tcagttcaag aaggctacct ctggcggcat ggttctggtc 1140
   atgagtctgt gggatgatta ctccgtcaac atgctgtggc tggactccac ctacccgaca 1200
   aacgccaccg gtacacccgg tgccgctcgc ggaacctgct ccaccagctc cggtgtccct 1260
   gctcaggtcg aatctcagtc tcccaacgcc aaggtcacct tctccaacat caagttcgga 1320
   cccattggca gcaccggcaa ccctagcggc ggcaaccctc ccggcggaaa cccgcctggc 1380
   accaccacca cccgccgccc agccactacc actggaagct ctcccggacc tacccagtct 1440
   cactacggcc agtgcggcgg tattggctac agcggcccca cggtctgcgc cagcggcaca 1500
   acttgccagg tcctgaaccc ttactactct cagtgcctgt aa 1542
<210> 12
   <211> 505
   <212> PRT
   <213> Artificial Sequence
<220>
   <223> Clone 225B11
<400> 12
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Ala Tyr Ala
                   85                  90                  95
   Ser Thr Tyr Gly Val Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Ser Val Asn Met Leu Trp Leu Asp Ser Thr Tyr Pro Thr
   385                 390                 395                 400
   Asn Ala Thr Gly Thr Pro Gly Ala Ala Arg Gly Thr Cys Ser Thr Ser
                   405                 410                 415
   Ser Gly Val Pro Ala Gln Val Glu Ser Gln Ser Pro Asn Ala Lys Val
               420                 425                 430
   Thr Phe Ser Asn Ile Lys Phe Gly Pro Ile Gly Ser Thr Gly Asn Pro
           435                 440                 445
   Ser Gly Gly Asn Pro Pro Gly Gly Asn Pro Pro Gly Thr Thr Thr Thr
       450                 455                 460
   Arg Arg Pro Ala Thr Thr Thr Gly Ser Ser Pro Gly Pro Thr Gln Ser
   465                 470                 475                 480
   His Tyr Gly Gln Cys Gly Gly Ile Gly Tyr Ser Gly Pro Thr Val Cys
                   485                 490                 495
   Ala Ser Gly Thr Thr Cys Gln Val Leu
               500                 505
<210> 13
   <211> 1545
   <212> DNA
   <213> Artificial Sequence
<220>
   <223> Clone 242D11
<400> 13
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg cctacgcgtc cacgtacgga     300
   gttaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctttacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctggcag ccgtcatcca acgatgcgaa cgcgggcacc     660
   ggaaaccacg gaagctgctg ctctgagatg gatatctggg aggccaactc catctccgag     720
   gctcttaccc cccacccttg cacgactgtc ggccaggaga tctgcgaggg tgatgggtgc       780
   ggcggaactt actccgataa cagatatggc ggcacttgcg atcccgatgg ctgcgactgg       840
   aacccatacc gcctgggcaa caccagcttc tacggccctg gctcaagctt taccctcgat       900
   accaccaaga aattgaccgt tgtcacccag ttcgagacgt cgggtgccat caaccgatac       960
   tatgtccaga atggcgtcac tttccagcag cccaacgccg agcttggtag ttactctggc      1020
   aacgagctca acgatgatta ctgcacagct gaggaggcag aattcggcgg atcctctttc      1080
   tcagacaagg gcggcctgac tcagttcaag aaggctacct ctggcggcat ggttctggtc      1140
   atgagtctgt gggatgatta ctacgccaac atgctgtggc tggactccac ctacccgaca      1200
   aacgagacct cctccacacc cggtgccgtg cgcggaagct gctccaccag ctccggtgtc      1260
   cctgctcagg tcgaatctca gtctcccaac gccaaggtca ccttctccaa catcaagttc      1320
   ggacccattg gcagcaccgg caaccctagc ggcggcaacc ctcccggcgg aaacccgcct      1380
   ggcaccacca ccacccgccg cccagccact accactggaa gctctcccgg acctacccag      1440
   tctcactacg gccagtgcgg cggtattggc tacagcggcc ccacggtctg cgccagcggc      1500
   acaacttgcc aggtcctgaa cccttactac tctcagtgcc tgtaa                      1545
<210> 14
   <211> 514
   <212> PRT
   <213> Artificial Sequence
<220>
   <223> Clone 242D11
<400> 14
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Ala Tyr Ala
                   85                  90                  95
   Ser Thr Tyr Gly Val Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Gln Pro Ser Ser Asn Asp Ala Asn Ala Gly Thr Gly Asn His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Glu Thr Ser Ser Thr Pro Gly Ala Val Arg Gly Ser Cys Ser Thr
                   405                 410                 415
   Ser Ser Gly Val Pro Ala Gln Val Glu Ser Gln Ser Pro Asn Ala Lys
               420                 425                 430
   Val Thr Phe Ser Asn Ile Lys Phe Gly Pro Ile Gly Ser Thr Gly Asn
           435                 440                 445
   Pro Ser Gly Gly Asn Pro Pro Gly Gly Asn Pro Pro Gly Thr Thr Thr
       450                 455                 460
   Thr Arg Arg Pro Ala Thr Thr Thr Gly Ser Ser Pro Gly Pro Thr Gln
   465                 470                 475                 480
   Ser His Tyr Gly Gln Cys Gly Gly Ile Gly Tyr Ser Gly Pro Thr Val
                   485                 490                 495
   Cys Ala Ser Gly Thr Thr Cys Gln Val Leu Asn Pro Tyr Tyr Ser Gln
               500                 505                 510
   Cys Leu
<210> 15
   <211> 1545
   <212> DNA
   <213> Artificial Sequence
<220>
   <223> Clone 453E8
<400> 15
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg actactcggg aacgtacgga     300
   attaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctttacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cacgactgtc ggccaggaga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc    1020
   aacgagctca acgatgatta ctgcacagct gaggaggcag aattcggcgg atcctctttc    1080
   tcagacaagg gcggcctgac tcagttcaag aaggctacct ctggcggcat ggttctggtc    1140
   atgagtctgt gggatgatta ctacgccaac atgctgtggc tggactccac ctacccgaca    1200
   aacgagacct cctccacacc cggtgccgtg cgcggaagct gctccaccag ctccggtgtc    1260
   cctgctcagg tcgaatctca gtctcccaac gccaaggtca ccttctccaa catcaagttc    1320
   ggacccattg gcagcaccgg caaccctagc ggcggcaacc ctcccggcgg aaacccgcct    1380
   ggcaccacca ccacccgccg cccagccact accactggaa gctctcccgg acctacccag    1440
   tctcactacg gccagtgcgg cggtattggc tacagcggcc ccacggtctg cgccagcggc    1500
   acaacttgcc aggtcctgaa cccttactac tctcagtgcc tgtaa                    1545
<210> 16
   <211> 514
   <212> PRT
   <213> Artificial Sequence
<220>
   <223> Clone 453E8
<400> 16
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Asp Tyr Ser
                   85                  90                  95
   Gly Thr Tyr Gly Ile Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Thr Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Glu Thr Ser Ser Thr Pro Gly Ala Val Arg Gly Ser Cys Ser Thr
                   405                 410                 415
   Ser Ser Gly Val Pro Ala Gln Val Glu Ser Gln Ser Pro Asn Ala Lys
               420                 425                 430
   Val Thr Phe Ser Asn Ile Lys Phe Gly Pro Ile Gly Ser Thr Gly Asn
           435                 440                 445
   Pro Ser Gly Gly Asn Pro Pro Gly Gly Asn Pro Pro Gly Thr Thr Thr
       450                 455                 460
   Thr Arg Arg Pro Ala Thr Thr Thr Gly Ser Ser Pro Gly Pro Thr Gln
   465                 470                 475                 480
   Ser His Tyr Gly Gln Cys Gly Gly Ile Gly Tyr Ser Gly Pro Thr Val
                   485                 490                 495
   Cys Ala Ser Gly Thr Thr Cys Gln Val Leu Asn Pro Tyr Tyr Ser Gln
               500                 505                 510
   Cys Leu
<210> 17
   <211> 1542
   <212> DNA
   <213> Artificial Sequence
<220>
   <223> Clone B
<400> 17
   atgtatcgga agttggccgt catctcggcc ttcttggcca cagctcgtgc tcagtcggcc      60
   tgcactctcc aatcggagac tcacccgcct ctgacatggc agaaatgctc gtctggtggc     120
   acgtgcactc aacagacagg ctccgtggtc atcgacgcca actggcgctg gactcacgct     180
   acgaacagca gcacgaactg ctacgatggc aacacttgga gctcgaccct atgtcctgac     240
   aacgagacct gcgcgaagaa ctgctgtctg gacggtgccg actactcggg aacgtacgga     300
   attaccacga gcggtaacag cctctccatt ggctttgtca cccagtctgc gcagaagaac     360
   gttggcgctc gcctttacct tatggcgagc gacacgacct accaggaatt caccctgctt     420
   ggcaacgagt tctctttcga tgttgatgtt tcgcagctgc cgtgcggctt gaacggagct     480
   ctttacttcg tgtccatgga cgcggatggt ggcgtgagca agtatcccac caacaccgct     540
   ggcgccaagt acggcacggg gtactgtgac agccagtgtc cccgcgatct gaagttcatc     600
   aatggccagg ccaacgttga gggctgggag ccgtcatcca acaacgcgaa cacgggcatt     660
   ggaggacacg gaagctgctg ctctgagatg gatatctggg aggccaactc catctccgag     720
   gctcttaccc cccacccttg cgacactccc ggccaggtga tctgcgaggg tgatgggtgc     780
   ggcggaactt actccgataa cagatatggc ggcacttgcg atcccgatgg ctgcgactgg     840
   aacccatacc gcctgggcaa caccagcttc tacggccctg gctcaagctt taccctcgat     900
   accaccaaga aattgaccgt tgtcacccag ttcgagacgt cgggtgccat caaccgatac     960
   tatgtccaga atggcgtcac tttccagcag cccaacgccg agcttggtag ttactctggc    1020
   aacgagctca acgatgatta ctgcacagct gaggaggcag aattcggcgg atcctctttc    1080
   tcagacaagg gcggcctgac tcagttcaag aaggctacct ctggcggcat ggttctggtc    1140
   atgagtctgt gggatgatta ctccgtcaac atgctgtggc tggactccac ctacccgaca    1200
   aacgccaccg gtacacccgg tgccgctcgc ggaacctgct ccaccagctc cggttcacct    1260
   aagaccgtcg aagccaactc tcccaacgcc aaggtcacct tctccaacat caagttcgga    1320
   cccattggca gcaccggcaa ccctagcggc ggcaaccctc ccggcggaaa cccgcctggc    1380
   accaccacca cccgccgccc agccactacc actggaagct ctcccggacc tacccagtct    1440
   cactacggcc agtgcggcgg tattggctac agcggcccca cggtctgcgc cagcggcaca    1500
   acttgccagg tcctgaaccc ttactactct cagtgcctgt aa                        1542
<210> 18
   <211> 513
   <212> PRT
   <213> Artificial Sequence
<220>
   <223> Clone B
<400> 18
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Glu Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Asp Tyr Ser
                   85                  90                  95
   Gly Thr Tyr Gly Ile Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Gln Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly His Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Asp Thr Pro Gly Gln Val Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asn Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Ser Val Asn Met Leu Trp Leu Asp Ser Thr Tyr Pro Thr
   385                 390                 395                 400
   Asn Ala Thr Gly Thr Pro Gly Ala Ala Arg Gly Thr Cys Ser Thr Ser
                   405                 410                 415
   Ser Gly Ser Pro Lys Thr Val Glu Ala Asn Ser Pro Asn Ala Lys Val
               420                 425                 430
   Thr Phe Ser Asn Ile Lys Phe Gly Pro Ile Gly Ser Thr Gly Asn Pro
           435                 440                 445
   Ser Gly Gly Asn Pro Pro Gly Gly Asn Pro Pro Gly Thr Thr Thr Thr
       450                 455                 460
   Arg Arg Pro Ala Thr Thr Thr Gly Ser Ser Pro Gly Pro Thr Gln Ser
   465                 470                 475                 480
   His Tyr Gly Gln Cys Gly Gly Ile Gly Tyr Ser Gly Pro Thr Val Cys
                   485                 490                 495
   Ala Ser Gly Thr Thr Cys Gln Val Leu Asn Pro Tyr Tyr Ser Gln Cys
               500                 505                 510
   Leu
<210> 19
   <211> 1545
   <212> DNA
   <213> Artificial Sequence
<220>
   <223> Clone 91D9
<400> 19
   atgtatcgga agttggccgt catctcggcc ttcttggcca cagctcgtgc tcagtcggcc 60
   tgcactctcc aatcggggac tcacccgcct ctgacatggc agaaatgctc gtctggtggc 120
   acgtgcactc aacagacagg ctccgtggtc atcgacgcca actggcgctg gactcacgct 180
   acgaacagca gcacgaactg ctacgatggc aacacttgga gctcgaccct atgtcctgac 240
   aacgagacct gcgcgaagaa ctgctgtctg gacggtgccg accgctcggg aacgtacgga 300
   attaccacga gcggtaacag cctctccatt ggctttgtca cccagtctgc gcagaagaac 360
   gttggcgctc gcctttacct tatggcgagc gacacgacct acctggaatt caccctgctt 420
   ggcaacgagt tctctttcga tgttgatgtt tcgcagctgc cgtgcggctt gaacggagct 480
   ctttacttcg tgtccatgga cgcggatggt ggcgtgagca agtatcccac caacaccgct 540
   ggcgccaagt acggcacggg gtactgtgac agccagtgtc cccgcgatct gaagttcatc 600
   aatggccagg ccaacgttga gggctgggag ccgtcatcca acaacgcgaa cacgggcatt 660
   ggaggacgcg gaagctgctg ctctgagatg gatatctggg aggccaactc catctccgag 720
   gctcttaccc cccacccttg catgactgtc ggccaggaga tctgcgaggg tgatgggtgc 780
   ggcggaactt actccgataa cagatatggc ggcacttgcg atcccgatgg ctgcgactgg 840
   aacccatacc gcctgggcaa caccagcttc tacggccctg gctcaagctt taccctcgat 900
   accaccaaga aattgaccgt tgtcacccag ttcgagacgt cgggtgccat caaccgatac 960
   tatgtccaga atggcgtcac tttccagcag cccaacgccg agcttggtag ttactctggc 1020
   aacgagctcg acgatgatta ctgcacagct gaggaggcag aattcggcgg atcctctttc 1080
   tcagacaagg gcggcctgac tcagttcaag aaggctacct ctggcggcat ggttctggtc 1140
   atgagtctgt gggatgatta ctacgccaac atgctgtggc tggactccac ctacccgaca 1200
   aacgagacct cctccacacc cggtgccgtg cgcggaagct gctccaccag ctccggtgtc 1260
   cctgctcagg tcgaatctca gtctcccaac gccaaggtca ccttctccaa catcaagctc 1320
   ggacccattg gcagcaccgg caaccctagc ggcggcaacc ctcccggcgg aaacccgcct 1380
   ggcaccacca ccacccgccg cccagccact accactggaa gctctcccgg acctacccag 1440
   tctcactacg gccagtgcgg cggtattggc tacagcggcc ccacggtctg cgccagcggc 1500
   acaacttgcc aggtcctgaa cccttactac tctcagtgcc tgtaa 1545
<210> 20
   <211> 514
   <212> PRT
   <213> Artificial Sequence
<220>
   <223> Clone 91D9
<400> 20
   Met Tyr Arg Lys Leu Ala Val Ile Ser Ala Phe Leu Ala Thr Ala Arg
   1               5                   10                  15
   Ala Gln Ser Ala Cys Thr Leu Gln Ser Gly Thr His Pro Pro Leu Thr
               20                  25                  30
   Trp Gln Lys Cys Ser Ser Gly Gly Thr Cys Thr Gln Gln Thr Gly Ser
           35                  40                  45
   Val Val Ile Asp Ala Asn Trp Arg Trp Thr His Ala Thr Asn Ser Ser
       50                  55                  60
   Thr Asn Cys Tyr Asp Gly Asn Thr Trp Ser Ser Thr Leu Cys Pro Asp
   65                  70                  75                  80
   Asn Glu Thr Cys Ala Lys Asn Cys Cys Leu Asp Gly Ala Asp Arg Ser
                   85                  90                  95
   Gly Thr Tyr Gly Ile Thr Thr Ser Gly Asn Ser Leu Ser Ile Gly Phe
               100                 105                 110
   Val Thr Gln Ser Ala Gln Lys Asn Val Gly Ala Arg Leu Tyr Leu Met
           115                 120                 125
   Ala Ser Asp Thr Thr Tyr Leu Glu Phe Thr Leu Leu Gly Asn Glu Phe
       130                 135                 140
   Ser Phe Asp Val Asp Val Ser Gln Leu Pro Cys Gly Leu Asn Gly Ala
   145                 150                 155                 160
   Leu Tyr Phe Val Ser Met Asp Ala Asp Gly Gly Val Ser Lys Tyr Pro
                   165                 170                 175
   Thr Asn Thr Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Glu Pro Ser Ser Asn Asn Ala Asn Thr Gly Ile Gly Gly Arg Gly
       210                 215                 220
   Ser Cys Cys Ser Glu Met Asp Ile Trp Glu Ala Asn Ser Ile Ser Glu
   225                 230                 235                 240
   Ala Leu Thr Pro His Pro Cys Met Thr Val Gly Gln Glu Ile Cys Glu
                   245                 250                 255
   Gly Asp Gly Cys Gly Gly Thr Tyr Ser Asp Asn Arg Tyr Gly Gly Thr
               260                 265                 270
   Cys Asp Pro Asp Gly Cys Asp Trp Asn Pro Tyr Arg Leu Gly Asn Thr
           275                 280                 285
   Ser Phe Tyr Gly Pro Gly Ser Ser Phe Thr Leu Asp Thr Thr Lys Lys
       290                 295                 300
   Leu Thr Val Val Thr Gln Phe Glu Thr Ser Gly Ala Ile Asn Arg Tyr
   305                 310                 315                 320
   Tyr Val Gln Asn Gly Val Thr Phe Gln Gln Pro Asn Ala Glu Leu Gly
                   325                 330                 335
   Ser Tyr Ser Gly Asn Glu Leu Asp Asp Asp Tyr Cys Thr Ala Glu Glu
               340                 345                 350
   Ala Glu Phe Gly Gly Ser Ser Phe Ser Asp Lys Gly Gly Leu Thr Gln
           355                 360                 365
   Phe Lys Lys Ala Thr Ser Gly Gly Met Val Leu Val Met Ser Leu Trp
       370                 375                 380
   Asp Asp Tyr Tyr Ala Asn Met Leu Trp Leu Asp Ser Thr Tyr Pro Thr
   385                 390                 395                 400
   Asn Glu Thr Ser Ser Thr Pro Gly Ala Val Arg Gly Ser Cys Ser Thr
                   405                 410                 415
   Ser Ser Gly Val Pro Ala Gln Val Glu Ser Gln Ser Pro Asn Ala Lys
                   420                 425                 430
   Val Thr Phe Ser Asn Ile Lys Leu Gly Pro Ile Gly Ser Thr Gly Asn
           435                 440                 445
   Pro Ser Gly Gly Asn Pro Pro Gly Gly Asn Pro Pro Gly Thr Thr Thr
       450                 455                 460
   Thr Arg Arg Pro Ala Thr Thr Thr Gly Ser Ser Pro Gly Pro Thr Gln
   465                 470                 475                 480
   Ser His Tyr Gly Gln Cys Gly Gly Ile Gly Tyr Ser Gly Pro Thr Val
                   485                 490                 495
   Cys Ala Ser Gly Thr Thr Cys Gln Val Leu Asn Pro Tyr Tyr Ser Gln
               500                 505                 510
   Cys Leu
<210> 21
   <211> 1566
   <212> DNA
   <213> Talaromyces stipitatus
<400> 21
   atgtatcgga agttggccgt catctcggcc ttcttggcca cagctcgtgc tcagcaggtc      60
   ggtacttccc aagcggaggt gcacccgtcc atgacatggc agagctgcac tgctggtggc     120
   agctgcacta ccaacaacgg caaggtggtc atcgacgcca actggcgctg ggtgcacaaa     180
   gtaggcgact acacgaactg ctacaccggc aacacttggg acaagaccct atgtcctgac     240
   gatgcaacct gcgcgtccaa ctgcgccctg gagggtgcca actaccagtc cacgtacgga     300
   gcgaccacga gcggtgacag cctccgcctc aactttgtca ccacctctca gcagaagaac     360
   attggctcgc gcctttacat gatgaaggac gacacgacct acgagatgtt caagctgctt     420
   aaccaggagt tcaccttcga tgttgatgtt tcgaacctgc cgtgcggctt gaacggagct     480
   ctctacttcg tggccatgga cgcggatggt ggcatgagca agtatcccac caacaaggct     540
   ggcgccaagt acggcacggg gtactgtgac agccagtgtc cccgcgatct gaagttcatc     600
   aatggccagg ccaacgttga gggctggcag ccgtcatcca acgatgcgaa cgcgggcacc     660
   ggaaaccacg gaagctgctg cgcggagatg gatatctggg aggccaactc catctccacg     720
   gctttcaccc cccacccttg cgacactccc ggccaggtga tgtgcaccgg tgatgcctgc     780
   ggcggaactt actcctccga cagatatggc ggcacttgcg atcccgatgg ctgcgacttc     840
   aactccttcc gccagggcaa caagaccttc tacggccctg gcatgaccgt cgataccaag     900
   agcaaattca ccgttgtcac ccagttcatc acggacgatg gcaccgccag cggcaccctc     960
   aaggagatca agcgattcta tgtccagaat ggcaaggtga tccccaactc ggagtcgacc    1020
   tggtccggcg tcggcggcaa ctccatcacc aacgattact gcacagctca gaagagcctg    1080
   ttcaaggacc agaacgtctt cgccaagcac ggcggcatgg agggcatggg cgctgctctc    1140
   gcccagggca tggttctggt catgagtctg tgggatgatc acgcggccaa catgctgtgg    1200
   ctggactcca actacccgac aactgcctct tcctccacac ccggtgtcgc ccgcggaacc    1260
   tgcgacatca gctccggtgt ccctgctgat gtcgaagcta accaccccga cgcctccgtc    1320
   gtctactcca acatcaaggt cggacccatt ggcagcacct tcaacagcgg cggctcgaac    1380
   cccggcggag gcaccaccac caccgcgaag ccaacgacga ccactaccac tgccggttct    1440
   cccggaggca ccggagtcgc tcagcactac ggccagtgcg gcggtaatgg ctggcagggc    1500
   cccacgacct gcgccagccc ttatacttgc cagaagctga acgactttta ctctcagtgc    1560
   ctgtaa                                                                1566
<210> 22
   <211> 521
   <212> PRT
   <213> Talaromyces stipitatus
<400> 22
   Met Tyr Arg Lys Leu Ala Val Ile Ser Ala Phe Leu Ala Thr Ala Arg
   1               5                   10                  15
   Ala Gln Gln Val Gly Thr Ser Gln Ala Glu Val His Pro Ser Met Thr
               20                  25                  30
   Trp Gln Ser Cys Thr Ala Gly Gly Ser Cys Thr Thr Asn Asn Gly Lys
           35                  40                  45
   Val Val Ile Asp Ala Asn Trp Arg Trp Val His Lys Val Gly Asp Tyr
       50                  55                  60
   Thr Asn Cys Tyr Thr Gly Asn Thr Trp Asp Lys Thr Leu Cys Pro Asp
   65                  70                  75                  80
   Asp Ala Thr Cys Ala Ser Asn Cys Ala Leu Glu Gly Ala Asn Tyr Gln
                   85                  90                  95
   Ser Thr Tyr Gly Ala Thr Thr Ser Gly Asp Ser Leu Arg Leu Asn Phe
               100                 105                 110
   Val Thr Thr Ser Gln Gln Lys Asn Ile Gly Ser Arg Leu Tyr Met Met
           115                 120                 125
   Lys Asp Asp Thr Thr Tyr Glu Met Phe Lys Leu Leu Asn Gln Glu Phe
       130                 135                 140
   Thr Phe Asp Val Asp Val Ser Asn Leu Pro Cys Gly Leu Asn Gly Ala
   145                 150                 155                 160
   Leu Tyr Phe Val Ala Met Asp Ala Asp Gly Gly Met Ser Lys Tyr Pro
                   165                 170                 175
   Thr Asn Lys Ala Gly Ala Lys Tyr Gly Thr Gly Tyr Cys Asp Ser Gln
               180                 185                 190
   Cys Pro Arg Asp Leu Lys Phe Ile Asn Gly Gln Ala Asn Val Glu Gly
           195                 200                 205
   Trp Gln Pro Ser Ser Asn Asp Ala Asn Ala Gly Thr Gly Asn His Gly
       210                 215                 220
   Ser Cys Cys Ala Glu Met Asp Ile Trp Glu Ala Asn Ser Ile Ser Thr
   225                 230                 235                 240
   Ala Phe Thr Pro His Pro Cys Asp Thr Pro Gly Gln Val Met Cys Thr
                   245                 250                 255
   Gly Asp Ala Cys Gly Gly Thr Tyr Ser Ser Asp Arg Tyr Gly Gly Thr
               260                 265                 270
   Cys Asp Pro Asp Gly Cys Asp Phe Asn Ser Phe Arg Gln Gly Asn Lys
           275                 280                 285
   Thr Phe Tyr Gly Pro Gly Met Thr Val Asp Thr Lys Ser Lys Phe Thr
       290                 295                 300
   Val Val Thr Gln Phe Ile Thr Asp Asp Gly Thr Ala Ser Gly Thr Leu
   305                 310                 315                 320
   Lys Glu Ile Lys Arg Phe Tyr Val Gln Asn Gly Lys Val Ile Pro Asn
                   325                 330                 335
   Ser Glu Ser Thr Trp Ser Gly Val Gly Gly Asn Ser Ile Thr Asn Asp
               340                 345                 350
   Tyr Cys Thr Ala Gln Lys Ser Leu Phe Lys Asp Gln Asn Val Phe Ala
           355                 360                 365
   Lys His Gly Gly Met Glu Gly Met Gly Ala Ala Leu Ala Gln Gly Met
       370                 375                 380
   Val Leu Val Met Ser Leu Trp Asp Asp His Ala Ala Asn Met Leu Trp
   385                 390                 395                 400
   Leu Asp Ser Asn Tyr Pro Thr Thr Ala Ser Ser Ser Thr Pro Gly Val
                   405                 410                 415
   Ala Arg Gly Thr Cys Asp Ile Ser Ser Gly Val Pro Ala Asp Val Glu
               420                 425                 430
   Ala Asn His Pro Asp Ala Ser Val Val Tyr Ser Asn Ile Lys Val Gly
           435                 440                 445
   Pro Ile Gly Ser Thr Phe Asn Ser Gly Gly Ser Asn Pro Gly Gly Gly
       450                 455                 460
   Thr Thr Thr Thr Ala Lys Pro Thr Thr Thr Thr Thr Thr Ala Gly Ser
   465                 470                 475                 480
   Pro Gly Gly Thr Gly Val Ala Gln His Tyr Gly Gln Cys Gly Gly Asn
                   485                 490                 495
   Gly Trp Gln Gly Pro Thr Thr Cys Ala Ser Pro Tyr Thr Cys Gln Lys
               500                 505                 510
   Leu Asn Asp Phe Tyr Ser Gln Cys Leu
           515                 520
<210> 23
   <211> 1557
   <212> DNA
   <213> Neosartorya fischeri
<400> 23
   atgtatcgga agttggccgt catctcggcc ttcttggcca cagctcgtgc tcagcagatt      60
   ggtaatctca ctgctgagac tcagccgtct ctgtcctggt ccacttgcac atctggtggc     120
   agctgcactt caaagagtgc ctccattact ttggacgcca actggcgctg ggtgcacagt     180
   gtcaacggca gcacgaactg ctacaccggc aacacttggg atacatccat ctgtgacact     240
   gatacttcct gcgcgcagga ctgcgctgtc gacggtgccg actactcggg aacgtacgga     300
   attaccacga gcggtaacag cctccgtctg aactttgtca ccggctctaa cgttggctcc     360
   cgcacttacc ttatggcgga taagacgcat taccagctct tcaacctgct taaccaggag     420
   ttcactttca cggttgatgc gtcgaccctg ccgtgcggct tgaacggagc tctctacttc     480
   gtgtccatgg acgcggatgg tggcgtgagc aagcaaccca acaacaaggc tggcgcccag     540
   tacggcgttg ggtactgtga cagccagtgt ccccgcgatc tgaagttcat cggcggccag     600
   gccaacgttg agggctggca accgtcatcc aacaactcca acacgggcct cggaaatcac      660
   ggaagctgct gcgcagagct tgatatctgg gaggccaact ccatctccga ggctcttacc      720
   ccccaccctt gcgatacttc gtcacagact gtatgcaccg gtgatgcctg cggcggaact      780
   tactccaatg acagatatgg cggcacttgc gatcccgatg gctgcgactt caacccatac      840
   cgcgttggcg tcaccgactt ctacggccct ggcatgacca ttgataccac caagcccgtg      900
   accgttgtca cccagttcgt cacgaatgac ggtacctcaa gcggtaccct ttctgagatc      960
   agacgatact atgtccagaa tggcaaggtc ttcgcacagc cctcttccaa gattgacgga     1020
   atttctggca acgccatcaa ctccgattac tgctccgctg agatttccac tttcggcgga     1080
   aacccttctt tcactaagca tggcggcctg gcaggtgtga gcacagctct taagaatggc     1140
   atggttctgg tcatgagtct gtgggatgat tactccgtca acatgctgtg gctggactcc     1200
   acctacccga caaacgccac cggtacaccc ggtgccgctc gcggaacctg ctccaccagc     1260
   tccggttcac ctaagaccgt cgaagccaac tctcccaacg cccatgtcat cttctccgat     1320
   atccgagttg gacccttgaa cagcaccttc agtggcagcg gcacttctac ccccggcgga     1380
   ggtagcagca ctaccaccag cccgggttcc actaccacta cccccggaag cggaagtgga     1440
   agtggagttg cttctcacta cggccagtgc ggcggtcagg gctggactgg ccccacgacc     1500
   tgcgccagcg gctttacttg cactgtcata aacccttact actctcagtg cctgtaa        1557
<210> 24
   <211> 518
   <212> PRT
   <213> Neosartorya fischeri
<400> 24
   Met Tyr Arg Lys Leu Ala Val Ile Ser Ala Phe Leu Ala Thr Ala Arg
   1               5                   10                  15
   Ala Gln Gln Ile Gly Asn Leu Thr Ala Glu Thr Gln Pro Ser Leu Ser
               20                  25                  30
   Trp Ser Thr Cys Thr Ser Gly Gly Ser Cys Thr Ser Lys Ser Ala Ser
           35                  40                  45
   Ile Thr Leu Asp Ala Asn Trp Arg Trp Val His Ser Val Asn Gly Ser
       50                  55                  60
   Thr Asn Cys Tyr Thr Gly Asn Thr Trp Asp Thr Ser Ile Cys Asp Thr
   65                  70                  75                      80
   Asp Thr Ser Cys Ala Gln Asp Cys Ala Val Asp Gly Ala Asp Tyr Ser
                   85                  90                  95
   Gly Thr Tyr Gly Ile Thr Thr Ser Gly Asn Ser Leu Arg Leu Asn Phe
   100 105 110
   Val Thr Gly Ser Asn Val Gly Ser Arg Thr Tyr Leu Met Ala Asp Lys
   115 120 125
   Thr His Tyr Gln Leu Phe Asn Leu Leu Asn Gln Glu Phe Thr Phe Thr
   130 135 140
   Val Asp Ala Ser Thr Leu Pro Cys Gly Leu Asn Gly Ala Leu Tyr Phe
   145 150 155 160
   Val Ser Met Asp Ala Asp Gly Gly Val Ser Lys Gln Pro Asn Asn Lys
   165 170 175
   Ala Gly Ala Gln Tyr Gly Val Gly Tyr Cys Asp Ser Gln Cys Pro Arg
   180 185 190
   Asp Leu Lys Phe Ile Gly Gly Gln Ala Asn Val Glu Gly Trp Gln Pro
   195 200 205
   Ser Ser Asn Asn Ser Asn Thr Gly Leu Gly Asn His Gly Ser Cys Cys
   210 215 220
   Ala Glu Leu Asp Ile Trp Glu Ala Asn Ser Ile Ser Glu Ala Leu Thr
   225 230 235 240
   Pro His Pro Cys Asp Thr Ser Ser Gln Thr Val Cys Thr Gly Asp Ala
   245 250 255
   Cys Gly Gly Thr Tyr Ser Asn Asp Arg Tyr Gly Gly Thr Cys Asp Pro
   260 265 270
   Asp Gly Cys Asp Phe Asn Pro Tyr Arg Val Gly Val Thr Asp Phe Tyr
   275 280 285
   Gly Pro Gly Met Thr Ile Asp Thr Thr Lys Pro Val Thr Val Val Thr
   290 295 300
   Gln Phe Val Thr Asn Asp Gly Thr Ser Ser Gly Thr Leu Ser Glu Ile
   305 310 315 320
   Arg Arg Tyr Tyr Val Gln Asn Gly Lys Val Phe Ala Gln Pro Ser Ser
   325 330 335
   Lys Ile Asp Gly Ile Ser Gly Asn Ala Ile Asn Ser Asp Tyr Cys Ser
   340 345 350
   Ala Glu Ile Ser Thr Phe Gly Gly Asn Pro Ser Phe Thr Lys His Gly
   355 360 365
   Gly Leu Ala Gly Val Ser Thr Ala Leu Lys Asn Gly Met Val Leu Val
   370 375 380
   Met Ser Leu Trp Asp Asp Tyr Ser Val Asn Met Leu Trp Leu Asp Ser
   385 390 395 400
   Thr Tyr Pro Thr Asn Ala Thr Gly Thr Pro Gly Ala Ala Arg Gly Thr
   405 410 415
   Cys Ser Thr Ser Ser Gly Ser Pro Lys Thr Val Glu Ala Asn Ser Pro
   420 425 430
   Asn Ala His Val Ile Phe Ser Asp Ile Arg Val Gly Pro Leu Asn Ser
   435 440 445
   Thr Phe Ser Gly Ser Gly Thr Ser Thr Pro Gly Gly Gly Ser Ser Thr
   450 455 460
   Thr Thr Ser Pro Gly Ser Thr Thr Thr Thr Pro Gly Ser Gly Ser Gly
   465 470 475 480
   Ser Gly Val Ala Ser His Tyr Gly Gln Cys Gly Gly Gln Gly Trp Thr
   485 490 495
   Gly Pro Thr Thr Cys Ala Ser Gly Phe Thr Cys Thr Val Ile Asn Pro
   500 505 510
   Tyr Tyr Ser Gln Cys Leu
   515
<210> 25
   <211> 33
   <212> DNA
   <213> Artificial Sequence
<220>
   <223> amorce forward exemple 5
<400> 25
   tccatcctcg agatgtatcg gaagttggcc gtc 33
<210> 26
   <211> 35
   <212> DNA
   <213> Artificial Sequence
<220>
   <223> amorce reverse exemple 5
<400> 26
   tccatcctcg agttacaggc actgagagta gtaag 35